# NEUE EUROPÄISCHE PATENTSCHRIFT

(11) **EP 0 888 436 B2**
(45) Veröffentlichungstag und Bekanntmachung des Hinweises auf die Entscheidung über den Einspruch: **18.09.2002**
(45) Hinweis auf die Patenterteilung: 21.06.2000
(21) Anmeldenummer: 97907074.5
(22) Anmeldetag: 06.03.1997
(51) Int. Cl.: C11D 3/39, C11D 3/28

(54) **FESTE ZUSAMMENSETZUNG AUS HETEROCYCLISCHEN VERBINDUNGEN UND/ODER OXIMESTERN UND INERTEN PORÖSEN TRÄGERMATERIALIEN**
SOLID COMPOSITION OF HETEROCYCLIC COMPOUNDS AND/OR OXIME ESTERS AND INERT POROUS CARRIER MATERIALS
COMPOSITION SOLIDE COMPRENANT DES COMPOSES HETEROCYCLIQUES ET/OU DES ESTERS D'OXIME ET DES MATERIAUX PORTEURS POREUX INERTES

(30) Priorität: 14.03.1996 DE 19609953
(43) Veröffentlichungstag der Anmeldung: 07.01.1999
(73) Patentinhaber: BASF AKTIENGESELLSCHAFT, 67056 Ludwigshafen (DE)
(72) Erfinder: OETTER, Günter, D-67227 Frankenthal (DE); WEHLAGE, Thomas, D-67346 Speyer (DE); KAPPES, Elisabeth, D-67117 Limburgerhof (DE); MÜLLER, Reinhard, D-67159 Friedelsheim (DE); BOECKH, Dieter, D-67117 Limburgerhof (DE); SCHÖNHERR, Michael, D-67227 Frankenthal (DE)
(86) Internationale Anmeldenummer: EP9701125
(87) Internationale Veröffentlichungsnummer: WO97033964

(56) Entgegenhaltungen:
- EP-A- 0 122 763
- EP-A- 0 267 046
- EP-B- 0 028 432
- DE-A- 3 003 351
- DE-A- 19 518 039
- FR-A- 2 398 798
- GB-A- 2 249 104

## Beschreibung

Die vorliegende Erfindung betrifft eine feste Zusammensetzung aus heterocyclischen Verbindungen mit cyclischer Carbamat-, Lactonoxy- oder Lactam-Struktur und/oder Oximestern und inerten porösen Trägermaterialien, deren innere Oberfläche einen bestimmten Wert aufweist. Weiterhin betrifft die Erfindung ein Verfahren zur Herstellung dieser festen Zusammensetzung sowie ihre Verwendung in Wasch-, Bleich- und Reinigungsmitteln, insbesondere als stabile Bleichaktivator-Komponente.

Eine Reihe von Kaltbleichaktivatoren, unter anderem auch ein Teil der nachfolgend definierten Verbindungen I und II, sind in reiner Form flüssig oder plastisch und lassen sich demzufolge mit den üblichen Hilfsstoffen und Konfektioniermethoden nur sehr schlecht in ein rieselfähiges Granulat oder Pulver überführen. Rieselfähige und nicht zu Verbackungen neigende Formulierungen sind jedoch Voraussetzung für einen Einsatz in Wasch- und teilweise in Reinigungsmittelformulierungen.

Übliche Trocknungsverfahren zur Erzeugung von Feststoffen aus Lösungen wie z.B. die Sprühtrocknung ergeben häufig für in reiner Form fest vorliegende Kaltbleichaktivatoren unbefriedigende Resultate, da diese Substanzen in der Regel sehr hygroskopisch sind und meist relativ niedrige Schmelzpunkte aufweisen. Ebensowenig sind Kristallisationsverfahren anwendbar, da diese Substanzen oft zu übersättigten Lösungen neigen, d.h. stark gehemmt und somit nicht kontrollierbar kristallisieren.

Aufgabe der vorliegenden Erfindung war es daher, eine lagerstabile feste, rieselfähige Form für Kaltbleichaktivatoren mit heterocyclischer Struktur bereitzustellen, insbesondere unter Mitverwendung von Hilfsstoffen, die im Stand der Technik für derartige Zwecke zahlreich beschrieben sind. Dabei sollten diese Mittel auch in ihrer konfektionierten Form in Bezug auf die Auflösungsgeschwindigkeit und die Wirksamkeit in der Waschflotte verbessert werden.

So sind beispielsweise aus der DE-A 27 33 849 (1) feste Kaltbleichaktivatorzubereitungen für Wasch- und Reinigungsmittel bekannt, wobei als Kaltbleichaktivatoren ausschließlich Acylverbindungen wie Diacetylmethylamin oder Diacetylbutylamin, genannt werden. Diese Kaltbleichaktivatoren werden mit Adsorbentien wie Kieselgur, Silicaten, Kieselsäuren oder Aluminiumoxid zusammengebracht. Über innere Oberfläche und mittlere Teilchengröße dieser Adsorbentien finden sich in (1) jedoch keine Angaben.

Die DE-A 34 44 960 (2) betrifft ein grobkörniges Adsorptionsmittel für flüssige und pastöse Wasch- und Reinigungsmittelformulierungen, welche auch unter anderem Bleichaktivatoren enthalten können. Die so erzeugten Körner aus Adsorptionsmittel und Wasch- bzw. Reinigungsmittelformulierung können noch mit feinteiligen Pulvern bestäubt oder oberflächlich beschichtet werden. Als solche Puderungsmittel eignen sich z. B. Zeolithe oder Kieselsäureaerogel mit einer Korngröße von 0,001 bis 0,1 mm, Angaben über deren innere Oberfläche werden jedoch nicht gemacht.

Aus der EP-A 170 386 (3) sind Bleichsysteme bekannt, die als Bleichagens unter anderem N-Acyl- oder N-Benzoyl-6-aminoperoxycapronsäure enthalten. Diese Bleichsysteme werden in Waschmittelformulierungen eingesetzt, die als zum Bleichsystem separat vorliegenden Gerüststoff (Builder) Natriumaluminiumsilicate, z. B. Zeolith A, enthalten.

Die EP-A 382 464 (4) lehrt ein Beschichtungs- oder Verkapselungsverfahren für feste Partikel oder flüssige Tropfen, beispielsweise Bleichmittel, mit Polyethylenglykolen, Polyethylenoxiden, Polyvinylpyrrolidon, oxidiertem Polyethylen oder ähnlichen Stoffen. Dabei werden als Formulierungshilfsmittel ("crumbling agents") feinteilige Substanzen wie Aerosil® 380 oder Aerosil R 972 zugegeben.

Die GB-A-2 249 104 beschreibt Bleichaktivatorzusammensetzungen, im denen ein Bleichaktivator auf ein Aluminosilikat, sowie andere gitter-förmige Strukturen aufgebracht wird. Es macht weder Einschränkungen bezüglich der einzusetzenden Aktivatoren, noch bezüglich der inneren Oberfläche der Trägersubstanzen.

Es wurde nun eine homogen aufgebaute feste Zusammensetzung aus im wesentlichen 5 bis 98 -Gew.-Teilen heterocyclischer Verbindungen der allgemeinen Formel I

R¹-X-L¹ (I)

in der
- L¹: für
(a) einen cyclischen Carbamatrest der Formel 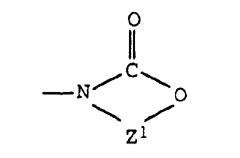
(b) einen Lactonoxyrest der Formel 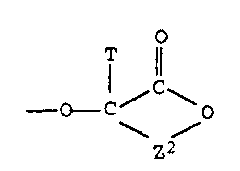 oder
(c) einen Lactamrest der Formel 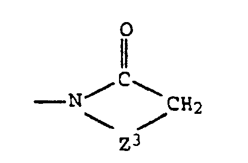 steht, wobei
- Z¹ bis Z³: 1,2-, 1,3-, 1,4- oder 1,5-Alkylengruppen mit 2 bis 20 C-Atomen, welche zusätzlich durch ein bis drei Hydroxylgruppen, C₁- bis C₄-Alkoxygruppen, Aminogruppen, C₁- bis C₄-Alkylaminogruppen, Di-C₁- bis C₄-alkylaminogruppen, Chloratome, Bromatome, Nitrogruppen, Cyanogruppen, Carboxylgruppen, Sulfogruppen, Carboxy-C₁- bis C₄-alkylgruppen, Carboxamidgruppen oder Phenyl-, Tolyl- oder Benzylreste funktionalisiert, wobei aromatische Kerne ihrerseits ebenfalls durch die genannten Reste substituiert sein können, oder durch ein oder zwei nicht benachbarte Sauerstoffatome, Aminogruppen, C₁- bis C₄-Alkylaminogruppen oder Carbonylgruppen unterbrochen sein können, bezeichnen und
- T: Wasserstoff oder C₁- bis C₄-Alkyl bedeutet,
- x: eine sauerstoffhaltige Gruppe der Formel 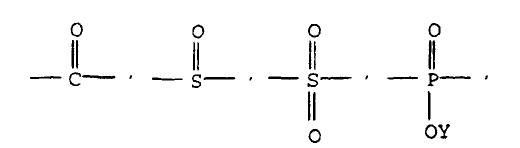 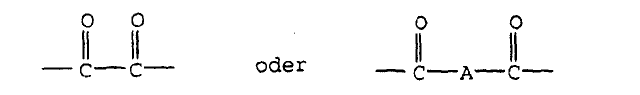 bedeutet, wobei
- Y: für Wasserstoff, Ammonium, welches gegebenenfalls durch organische Reste substituiert sein kann, oder C₁- bis C₄-Alkyl steht und
- A: eine chemische Bindung oder eine C₁- bis C₁₈-Alkylengruppe, eine C₂- bis C₁₈-Alkenylengruppe, eine C₅- bis C₃₂-Cycloalkylengruppe, eine C₇- bis C₃₀-Aralkylengruppe, eine C₆- bis C₁₈-Arylengruppe oder eine C₃- bis C₁₈-Heteroarylengruppe bezeichnet, wobei aliphatische Struktureinheiten zusätzlich durch ein bis fünf Hydroxylgruppen, C₁- bis C₄-Alkoxygruppen, Aminogruppen, C₁- bis C₄-Alkylaminogruppen, Di-C₁- bis C₄-alkylaminogruppen, Chloratome, Bromatome, Nitrogruppen, Cyanogruppen, Carboxylgruppen, Sulfogruppen, Carboxy-C₁- bis C₄-alkylgruppen, Carboxamidgruppen oder Phenyl-, Tolyl- oder Benzylreste funktionalisiert, wobei aromatische, cycloaliphatische und heteroaromatische Struktureinheiten ebenfalls durch die genannten Reste substituiert sein können, oder durch ein bis acht nicht benachbarte Sauerstoffatome, Aminogruppen, C₁- bis C₄-Alkylaminogruppen oder Carbonylgruppen unterbrochen sein können, und
- R¹: folgende Bedeutung aufweist:
C₁- bis C₃₀-Alkyl, C₂- bis C₃₀-Alkenyl, C₅- bis C₁₈-Cycloalkyl, C₇- bis C₁₈-Aralkyl, C₆- bis C₁₈-Aryl oder C₃- bis C₁₈-Heteroaryl, wobei aliphatische Reste zusätzlich durch ein bis fünf Hydroxylgruppen, C₁- bis C₄-Alkoxygruppen, Aminogruppen, C₁- bis C₄-Alkylaminogruppen, Di-C₁- bis C₄-alkylaminogruppen, Chloratome, Bromatome, Nitrogruppen, Cyanogruppen, Carboxylgruppen, Sulfogruppen, Carboxy-C₁bis C₄-alkylgruppen, Carboxamidgruppen oder Phenyl-, Tolyl- oder Benzylreste funktionalisiert, wobei aromatische, cycloaliphatische und heteroaromatische Struktureinheiten ebenfalls durch die genannten Reste substituiert sein können, oder durch ein bis acht nicht benachbarte Sauerstoffatome, Aminogruppen, C₁- bis C₄-Alkylaminogruppen oder Carbonylgruppen unterbrochen sein können,
   oder
einen heterocyclischen Rest L¹,
und/oder Oximestern der allgemeinen Formel II 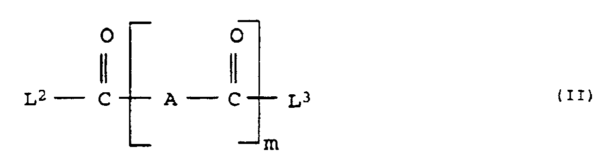 in der
- L²: für eine Oxim-Gruppierung der Formel 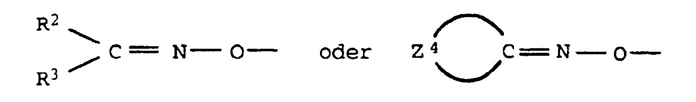 steht, wobei
- R² und R³: Wasserstoff, C₁- bis C₃₀-Alkyl, C₂- bis C₃₀-Alkenyl, C₅-bis C₁₈-Cycloalkyl, C₇- bis C₁₈-Aralkyl oder C₆bis C₁₈-Aryl oder C₃- bis C₁₈-Heteroaryl, wobei aliphatische Reste zusätzlich durch ein bis fünf Hydroxylgruppen, C₁- bis C₄-Alkoxygruppen, Aminogruppen, C₁- bis C₄-Alkylaminogruppen, Di-C₁- bis C₄alkylaminogruppen, Chloratome, Bromatome, Nitrogruppen, Cyanogruppen, Carboxylgruppen, Sulfogruppen, Carboxy-C₁- bis C₄-alkylgruppen, Carboxamidgruppen oder Phenyl-, Tolyl- oder Benzylreste funktionalisiert, wobei aromatische, cycloaliphatische und heteroaromatische Struktureinheiten ebenfalls durch die genannten Reste substituiert sein können, oder durch ein bis acht nicht benachbarte Sauerstotfatome, Aminogruppen, C₁-bis C₄-Alkylaminogruppen oder Carbonylgruppen unterbrochen sein können, bedeuten und
- Z⁴: 1,3-, 1,4-, 1,5-, 1,6-, 1,7- oder 1,8-Alkylengruppen mit 3 bis 30 C-Atomen, welche zusätzlich durch eir bis fünf Hydroxylgruppen, C₁- bis C₄-Alkylaminogruppen, Di-C₁- bis C₄-alkylaminogruppen, Chloratome Bromatome, Nitrogruppen, Cyanogruppen, Carboxylgruppen, Sulfogruppen, Carboxy-C₁- bis C₄-alkylgruppen, Carboxamidgruppen oder Phenyl-, Tolyl- oder Benzylreste funktionalisiert, wobei aromatische Kerne ihrerseits ebenfalls durch die genannten Reste substituiert sein können, oder durch ein oder zwei nicht benachbarte Sauerstoffatome, Aminogruppen, C₁- bis C₄-Alkylaminogruppen oder Carbonylgruppen unterbrochen sein können, bezeichnet,
- L³: für den Rest R¹, eine zweite Oxim-Gruppierung L² oder für
(a) einen Carbonesterrest der Formel 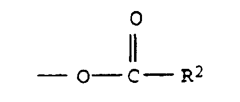
(b) einen Carbonamidrest der Formel 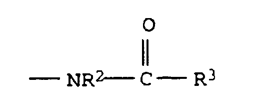
(c) einen Phenolatrest der Formel 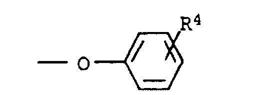
(d) einen Vinyloxyrest der Formel

   -O-CR²=CHR³
(e) einen Sulfonamidrest der Formel 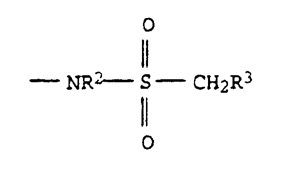
(f) einen Imidazolrest der Formel 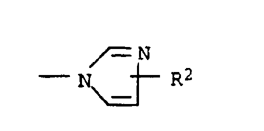
(g) einen Amidolactamrest der Formel 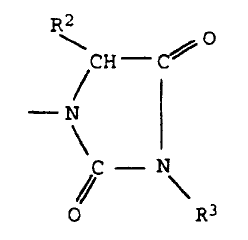
(h) einen cyclischen Carbamatrest der Formel 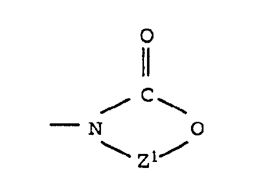
(j) einen Lactonoxyrest der Formel 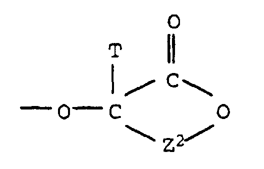 oder
(k) einen Lactamrest der Formel 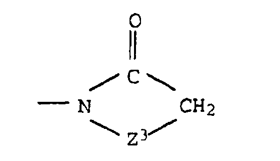 steht, wobei
- R¹, R², R^{3,} T, Z¹ bis Z³ und A: die oben genannten Bedeutungen haben,
- R⁴: Wasserstoff, eine Carbonsäuregruppe, eine Sulfonsäuregruppe, eine Phosphonsäuregruppe oder deren Alkalimetall -oder Ammoniumsalz bedeutet und
- m: für die Zahl 0 oder 1 steht,
und 2 bis 95 Gew.-Teilen inerter poröser Trägermaterialien mit einer inneren Oberfläche von 10 bis 500 m²/g, erhältlich durch gleichmäßiges Einziehen der heterocyclischen Verbindungen I bzw. der Oximester II und gegebenenfalls weiterer Hilfsstoffe in die inneren Bereiche der Trägermaterial-Körner und dortiges gleichmäßiges Verteilen, dergestalt daß kein merkliches Konzentrationsgefälle der einziehenden Substanzen zwischen Innerem und äußerer Oberfläche der Körner oder Körner-Aggregate vorliegt, gefunden.

Die Variablen Z¹ bis Z³ in den heterocyclischen Systemen (a) bis (c) können vor allem C₂- bis C₁₀-Alkylengruppierungen der folgenden Struktur bedeuten:

―CH₂CH₂―,

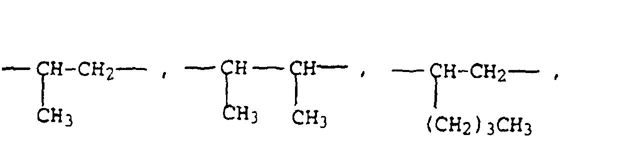

―CH₂CH₂CH₂―,

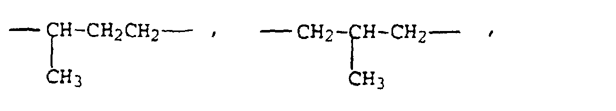

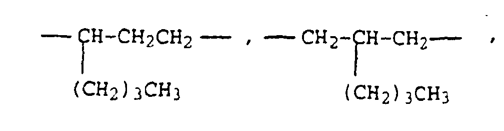

―CH₂CH₂CH₂CH₂―,

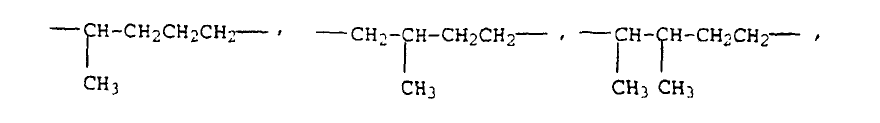

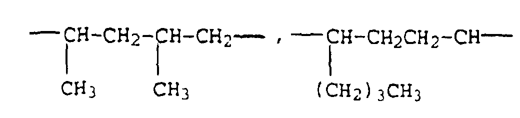

oder

―CH₂CH₂CH₂CH₂CH₂―,

wobei bei unsymmetrischen Alkylengruppierungen prinzipiell beide Einbaumöglichkeiten in die Ringe möglich sind. Die Variablen Z¹ bis Z³ können wie angegeben funktionalisiert oder unterbrochen sein.

Typische Beispiele für das Brückenglied A sind die folgenden:
- als lineare oder verzweigte C₁- bis C₁₈-Alkylengruppe, insbesondere C₆- bis C₁₂-Alkylongruppe, können Methylen, 1,2-Ethylen, 1,1-Ethylen, 1,3-Propylen, 1,2-Propylen, 1,1-Propylen, 2,2-Propylen, 1,4-Butylen, 1,2-Butylen, 2,3-Butylen, Pentamethylen, 3-Methyl-1,5-pentylen, Hexamethylen, Heptamethylen, Octamethylen, Nonamethylen, Decamethylen, Undecamethylen, Dodecamethylen, Tetradecamethylen, Hexadecamethylen oder Octadecamethylen auftreten;
- als lineare oder verzweigte C₂- bis C₁₈-Alkenylengruppe, insbesondere C₆- bis C₁₂-Alkenylengruppe, können Brückenglieder mit ein, zwei oder drei olefinischen Doppelbindungen oder auch acetylenischen Doppelbindungen auftreten, z.B. 1,2-Ethenylen, 1,3-Propenylen, 1,4-But-2-enylen, 1,6-Hex-3-enylen, 1,8-Oct-4-enylen oder 1,12-Dodec-6-enylen;
- als C₅ bis C₃₂-Cycloalkylengruppen, insbesondere C₅- bis C₁₀-Cycloalkylengruppen, eignen sich 1,2- oder 1,3-Cyclopentylen, 1,2-, 1,3-oder 1,4-Cyclohexylen, 1,2-, 1,3-oder 1,4-Cycloheptylen, 1,2-, 1,3-, 1,4- oder 1,5-Cyclooctylen oder Gruppierungen der Formel 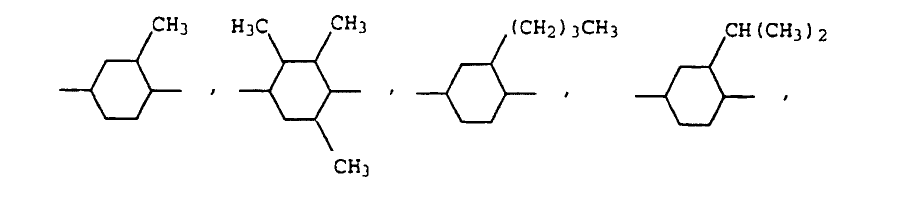 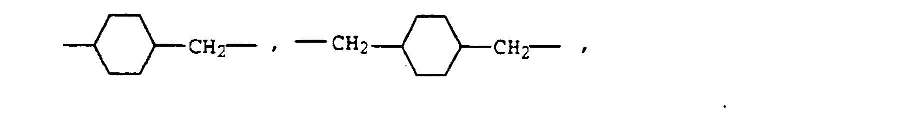 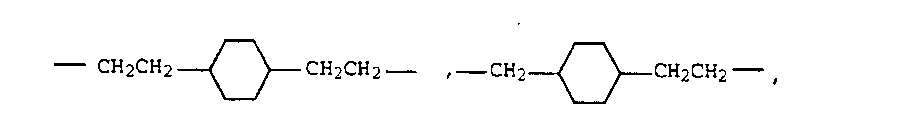 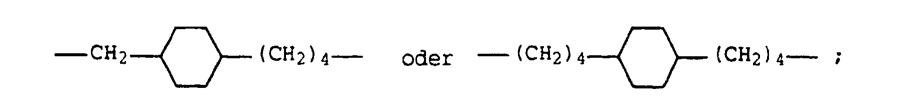
- als C₇- bis C₃₀-Aralkylengruppen, insbesondere gegebenenfalls alkylsubstituierte C₇- bis C₂₂-Phenylalkylen- und -Diphenylalkylengruppen, kommen in Betracht Gruppierungen der Formel 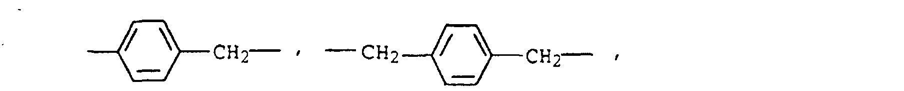 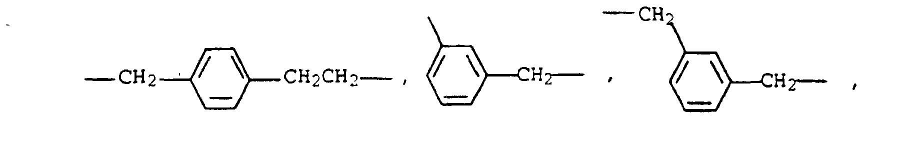 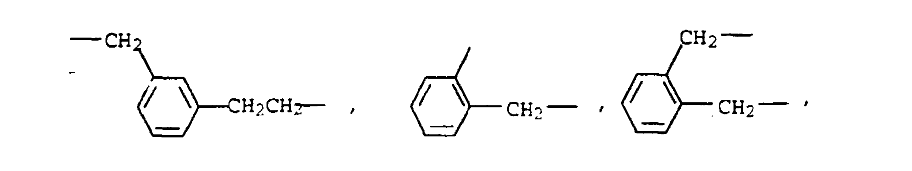 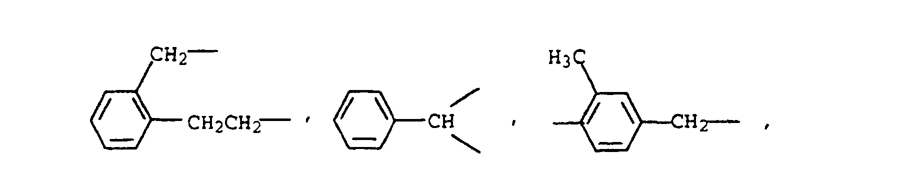 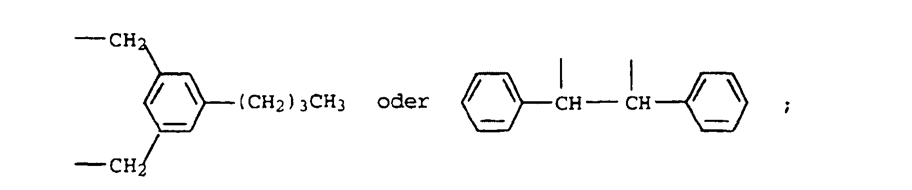
- als C₆- bis C₁₈-Arylengruppen, insbesondere gegebenenfalls alkylsubstituierte Phenylen-, Bisphenylen- oder Naphthylengruppen, eignen sich vor allem 1,4-, 1,3- und 1,2-Phenylen, aber auch Gruppierungen der Formel 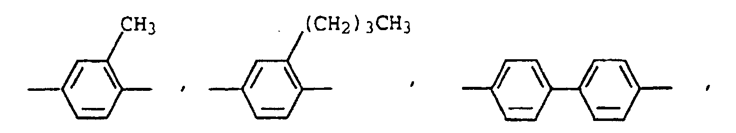 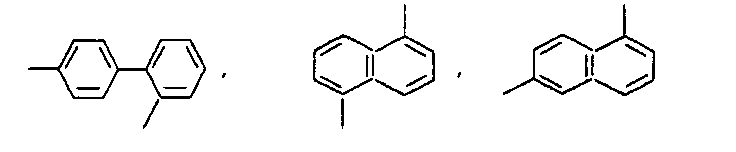 oder 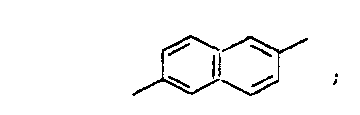
- als C₃- bis C₁₈-Heteroarylengruppen, insbesondere fünf- oder sechsgliedrige C₃- bis C₁₂-Heteroarylengruppen mit ein oder zwei Heteroatomen aus der Gruppe Stickstoff, Sauerstoff und Schwefel kommen in Betracht Gruppierungen der Formel 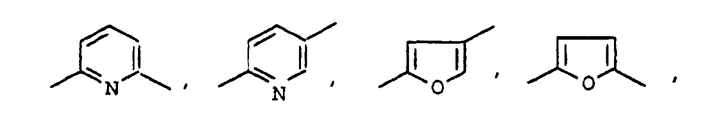 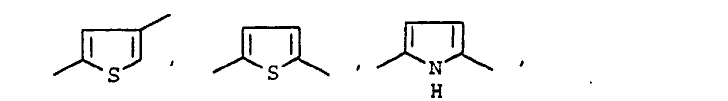 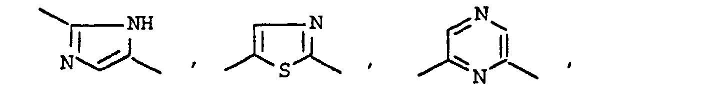 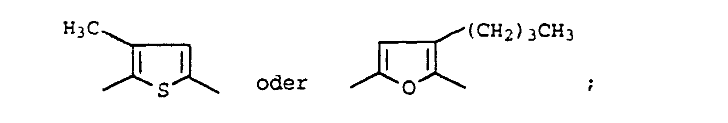
- als durch Sauerstoff oder Amino-Gruppierungen, insbesondere NH- oder N(CH₃)-Gruppierungen, unterbrochene Strukturen kommen beispielsweise folgende Strukturen in Betracht:

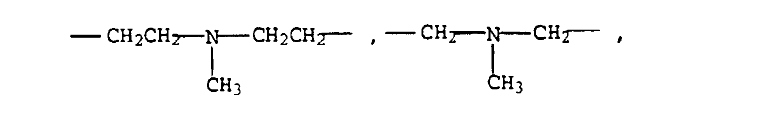

   ―CH₂―O―CH₂―, ―CH₂CH₂―O―CH₂― ,

   ―CH₂CH₂―O―CH₂CH₂―,

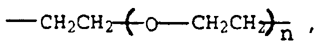

   ―(CH₂)₄―O―(CH₂)₄―,

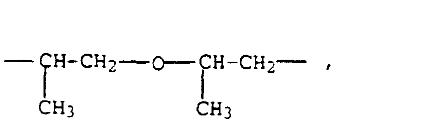

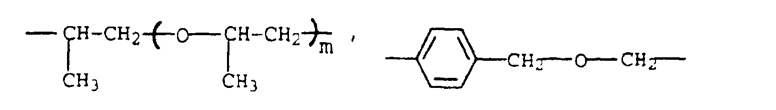

   oder 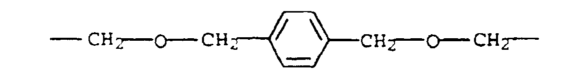 mit n = 2 bis 8 und m = 2 bis 5.

Für den Rest R¹ kommen folgende Bedeutungen in Betracht:
- als C₁- bis C₃₀-Alkylgruppe eignen sich beispielsweise Methyl, Ethyl, n-Propyl, iso-Propyl, n-Butyl, iso-Butyl, sek.-Butyl, tert.-Butyl, n-Pentyl, iso-Pentyl, sek.-Pentyl, tert.-Pentyl, neo-Pentyl, n-Hexyl, n-Heptyl, n-Octyl, 2-Ethylhexyl, n-Nonyl, iso-Nonyl, n-Decyl, n-Undecyl, n-Dodecyl, n-Tridecyl, iso-Tridecyl, n-Tetradecyl, n-Pentadecyl, n-Hexadecyl, n-Heptadecyl, n-Octadecyl, n-Nonadecyl oder n-Eicosyl; bevorzugt werden C₆- bis C₁₈-Alkylgruppen, vor allem C₈- bis C₁₂-Alkylgruppen;
- als C₂- bis C₃₀-Alkenylgruppe kommen beispielsweise Vinyl, Allyl, 2-Methylprop-2-enyl oder der entsprechende von Ölsäure, Linolsäure oder Linolensäure abgeleitete Rest in Frage; bevorzugt werden C₁₆- bis C₂₂-Alkenylgruppen;
- als C₅- bis C₁₈-Cycloalkylgruppe eignen sich vor allem C₅- bis C₁₀-Cycloalkylgruppen, z.B. Cyclopentyl, Cyclohexyl, 2-, 3- oder 4-Methylcyclohexyl, 2,3-, 2,4-, 2,5- oder 2,6-Dimethylcyclohexyl, Cycloheptyl oder Cyclooctyl;
- als C₇- bis C₁₈-Aralkyl-, insbesondere C₇- bis C₁₂-Aralkylgruppe kommen insbesondere alkylsubstituierte Phenylalkylgruppen in Frage, z.B. Benzyl; 2-, 3- oder 4-Methylbenzyl, 2-Phenylethyl, 3-Phenylpropyl, 4-Phenylbutyl, 2-, 3- oder 4-Ethylbenzyl, 3- oder 4-Isopropylbenzyl oder 3- oder 4-Butylbenzyl;
- als C₆- bis C₁₈-Arylgruppe eignen sich beispielsweise Phenyl, 2-, 3- oder 4-Bisphenyl, α- oder β-Naphthyl, 2-, 3-oder 4-Methylphenyl, 2-, 3- oder 4-Ethylphenyl, 3- oder 4-lsopropylphenyl, 3- oder 4-Butylphenyl oder 3- oder 4-(2'-Ethylhexyl)phenyl; bevorzugt werden C₆- bis C₁₄-Arylgruppen, insbesondere Phenyl und alkylsubstituiertes Phenyl;
- als C₃- bis C₁₈-Heteroarylgruppe kommen insbesondere fünf- oder sechsgliedrige C₃- bis C₁₂-Heteroarylgruppen mit ein oder zwei Heteroatomen aus der Gruppe Stickstoff, Sauerstoff und Schwefel in Frage, Beispiele hierfür sind: 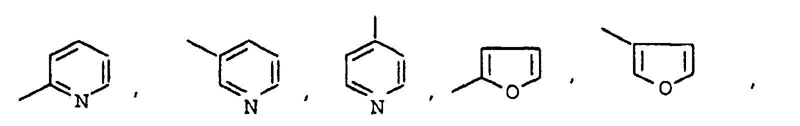 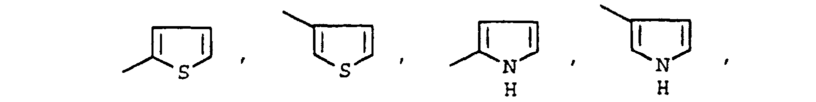 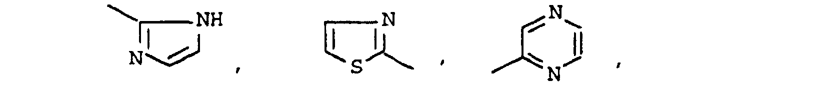 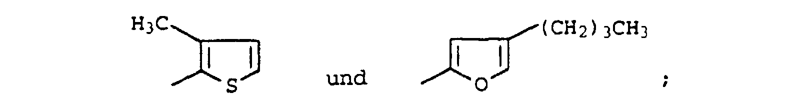
- als durch Sauerstoff oder Amino-Gruppierungen, insbesondere NH- oder N(CH₃)-Gruppierungen, unterbrochene aliphatische Reste kommen beispielsweise folgende Strukturen in Betracht:

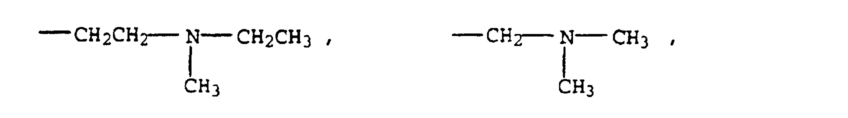

   ―CH₂―O―CH₃, ―CH₂CH₂―O―CH₃, ―CH₂CH₂―O―CH₂CH₃,

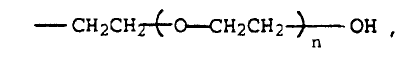

   ―(CH₂)₄―O―CH₃,

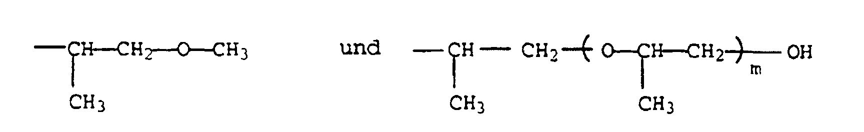

   mit n = 2 bis 8 und m = 2 bis 5.

Die oben definierten Variablen Z¹ bis Z³, A und R¹ können zusätzlich durch die angegebenen Gruppen funktionalisiert sein. Dabei bedeuten C₁- bis C₄-Alkoxygruppen insbesondere Methoxy, Ethoxy, n-Propoxy- iso-Propoxy, n-Butoxy, iso-Butoxy, sec-Butoxy oder tert.-Butoxy. Als Amino-Gruppierungen werden -NH₂, -NH(CH₃), -NH(CH₂CH₃), -N(CH₃)₂ und -N(CH₂CH₃)₂ bevorzugt.
Carboxy-C₁- bis C₄-alkylgruppen sind beispielsweise Carboxymethyl, Carboxyethyl, Carboxypropyl, Carboxybutyl oder Carboxy-tert.-butyl.

Die sauerstoffhaltige Gruppe X, bei der ein oder zwei Sauerstoffatome durch eine Doppelbindung an Kohlenstoff-, Schwefel- oder Phosphoratome gebunden sind, also Carbonyl- oder Heterocarbonyl-Funktionalitäten darstellen, bedeutet vorzugsweise

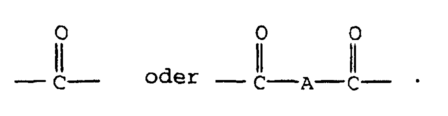

Für-den Fall R¹ = L¹ sind die beiden an die Gruppe X gebundenen heterocyclischen Reste L¹ vorzugsweise gleich.

Besonders bevorzugt wird eine feste Zusammensetzung, bei der der Rest R¹ in den heterocyclischen Verbindungen I C₆- bis C₁₃-Alkyl, C₆- bis C₁₈-Alkenyl, C₇- bis C₁₂-Aralkyl, Phenyl oder alkylsubstituiertes Phenyl mit insgesamt bis zu 14 C-Atomen oder einen zweiten heterocyclischen Rest L¹, der die gleiche Struktur wie der ersten heterocyclische Rest L¹ aufweist, bedeutet. Im Hinblick auf die bevorzugte Verwendung der heterocyclischen Verbindungen I als Kaltbleichaktivatoren in Wasch-, Bleich- und Reinigungsmitteln hat es sich als günstig herausgestellt, wenn R¹ einen langkettigen oder voluminösen Rest mit entsprechender Hydrophobie darstellt.

Folgende Strukturtypen der heterocyclischen Verbindungen I werden bevorzugt eingesetzt:
(1) N-Acyloxazolidone der Formel 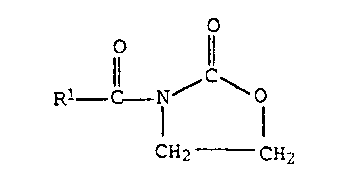
(2) N-Acyl-1,3-tetrahydrooxazinone der Formel 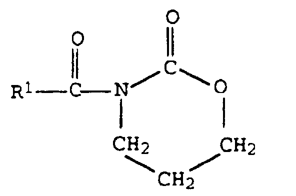
(3) Acyloxy-γ-butyrolactone der Formel 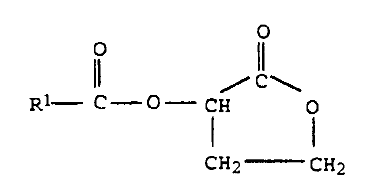
(4) Acyloxy-γ-valerolactone der Formel 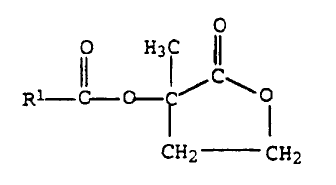
(5) O-Acylpantolactone der Formel 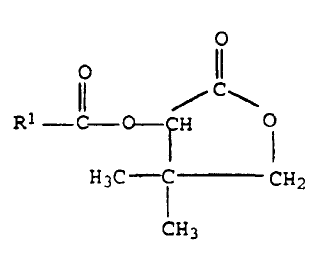
(6) Acyloxy-δ-valerolactone der Formel 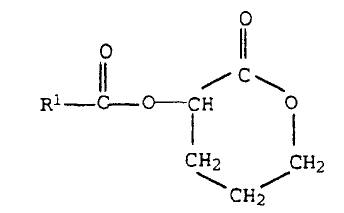
(7) Acyloxy-ε-caprolactone der Formel 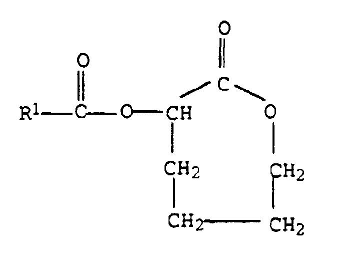
(8) Acyl-γ-butyrolactame der Formel 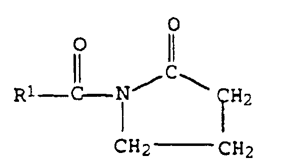
(9) N-Acyl-δ-valerolactame der Formel 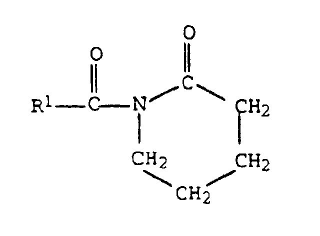
(10) N-Acyl-ε-caprolactame der Formel 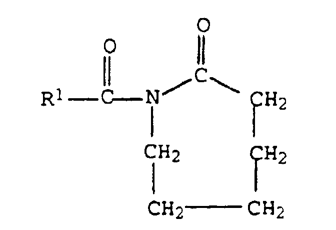
(11) N,N'-Carbonylbisoxazolidone der Formel 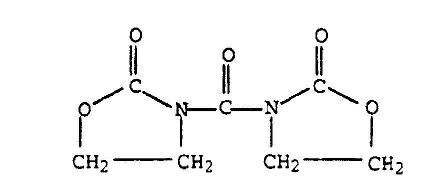
(12) N,N'-Carbonylbis-1,3-tetrahydrooxazinone der Formel 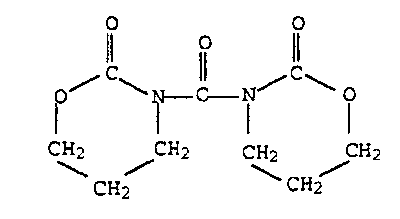
(13) Kohlensäurebis-γ-butyrolactone der Formel 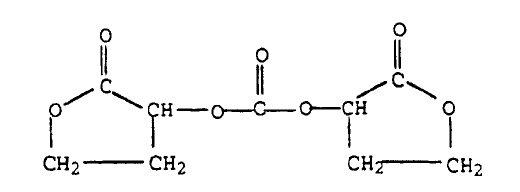
(14) Kohlensäurebis-γ-valerolactone der Formel 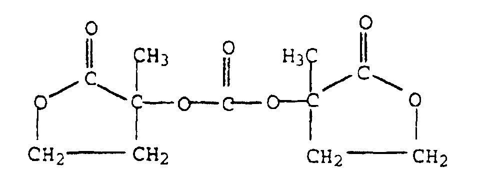
(15) O,O'-Carbonylbispantolactone der Formel 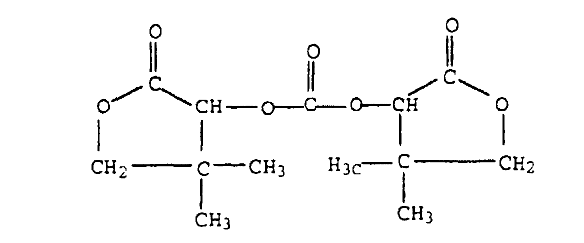
(16) Kohlensäurebis-δ-valerolactone der Formel 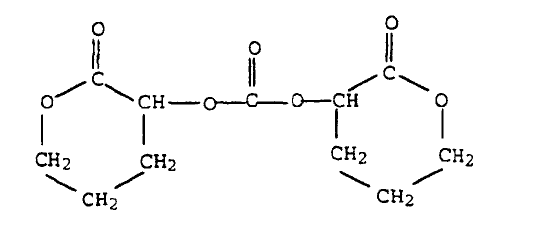
(17) Kohlensäurebis-ε-caprolactone der Formel 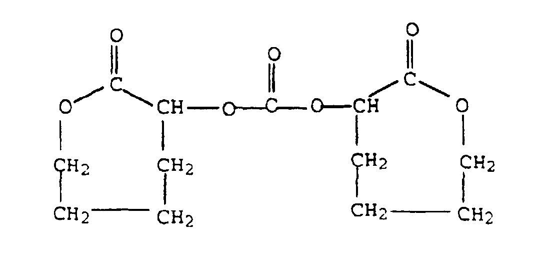
(18)N,N'-Carbonylbis-γ-butyrolactame der Formel 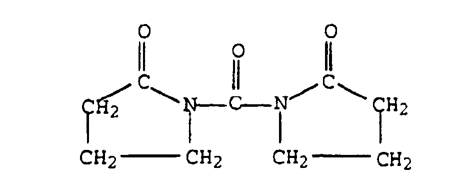
(19)N,N'-Carbonylbis-δ-valerolactame der Formel 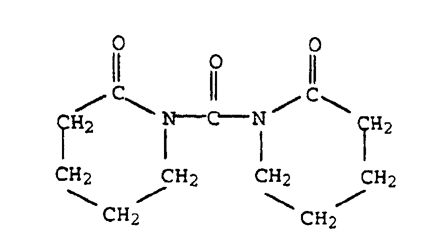
(20)N,N'-Carbonylbis-ε-caprolactame der Formel 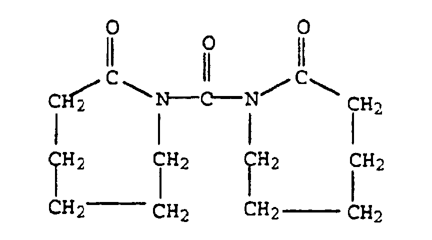
(21) verdoppelte N-Acyloxazolidone der Formel 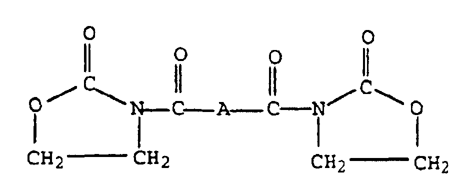
(22) verdoppelte N-Acyl-1,3-tetrahydrooxazinone der Formel 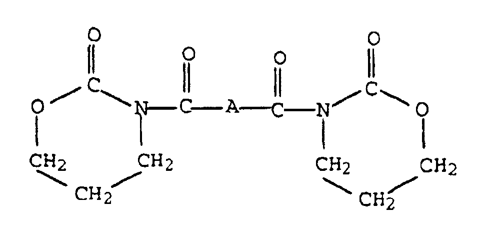
(23) verdoppelte Acyloxy-γ-butyrolactone der Formel 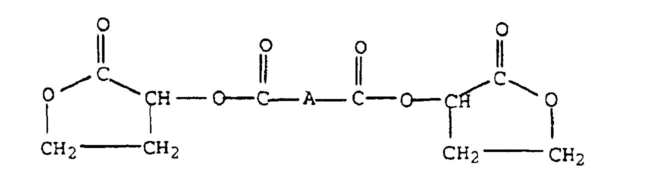
(24) verdoppelte Acyloxy-γ-valerolactone der Formel 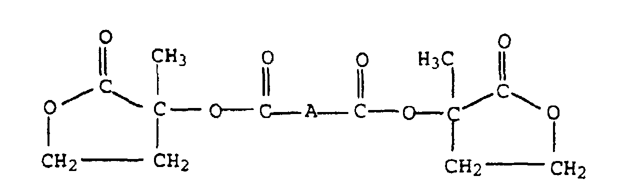
(25) verdoppelte O-Acylpantolactone der Formel 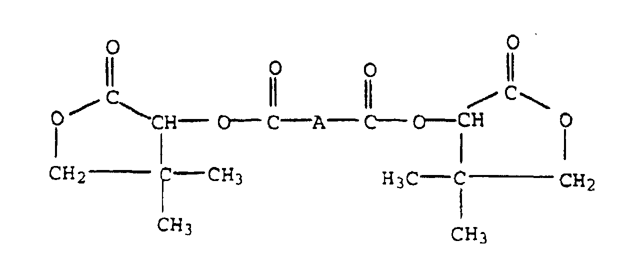
(26) verdoppelte Acyloxy-δ-valerolactone der Formel 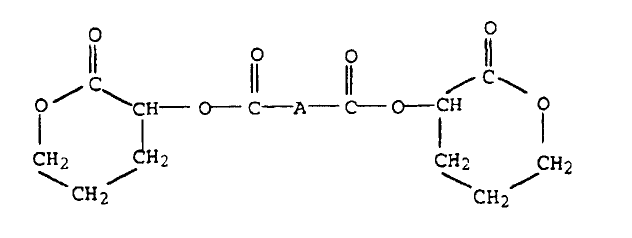
(27) verdoppelte Acyloxy-ε-caprolactone der Formel 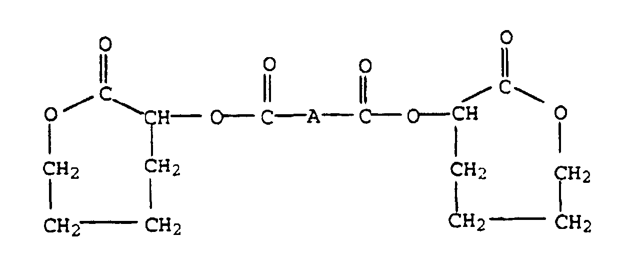
(28) verdoppelte Acyl-γ-butyrolactame der Formel 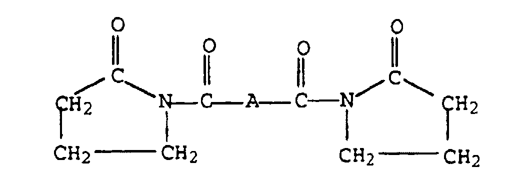
(29)verdoppelte Acyl-δ-valerolactame der Formel 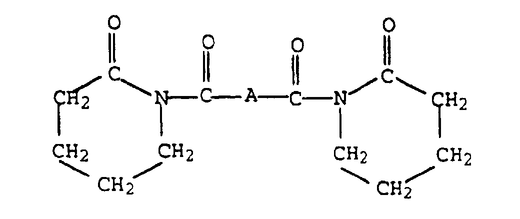
(30) verdoppelte N-Acyl-ε-caprolactame der Formel

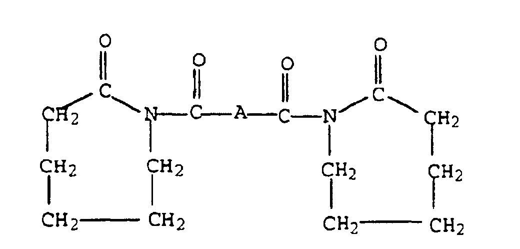

Der Rest R¹ in den Strukturtypen (1) bis (10) steht insbesondere für n-Hexyl, n-Heptyl, n-Octyl, n-Nonyl, n-Decyl, n-Undecyl, n-Dodecyl, Cyclohexyl, Benzyl, Phenyl oder 2-, 3- oder 4-Methylphenyl.

Das Brückenglied A in den Strukturtypen (21) bis (30) steht insbesondere für Hexamethylen, Octamethylen, Decamethylen, Dodecamethylen, 1,3- oder 1,4-Cyclohexylen oder 1,4-, 1,3- oder 1,2-Phenylen; von besonderem Interesse ist für A 1,4-Phenylen (abgeleitet von Terephthalsäure).

Für die Reste R² und R³ in den Oximestern II, die gleich oder verschieden sein können, kommen neben Wasserstoff folgende Bedeutungen in Betracht:
- als lineare oder verzweigte C₁- bis C₃₀-Alkylgruppen eignen sich beispielsweise Methyl, Ethyl, n-Propyl, iso-Propyl, n-Butyl, iso-Butyl, sek.-Butyl, tert.-Butyl, n-Pentyl, iso-Pentyl, sek.-Pentyl, tert.-Pentyl, neo-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl; bevorzugt werden C₁- bis C₁₂-Alkylgruppen, insbesondere C₁bis C₄-Alkylgruppen;
- als lineare oder verzweigte C₂- bis C₃₀-Alkenylgruppen kommen beispielsweise Vinyl, Allyl, 2-Methylprop-2-enyl oder der entsprechende von Ölsäure, Linolsäure oder Linolensäure abgeleitete Rest in Frage; bevorzugt werden C₂- bis C₆-Alkenyl- sowie C₁₆- bis C₂₂-Alkenylgruppen;
- als C₅- bis C₁₈-Cycloalkylgruppen eignen sich vor allem C₅- bis C₁₀-Cycloalkylgruppen, z.B. Cyclopentyl, Cyclohexyl, 2-, 3- oder 4-Methylcyclohexyl, 2,3-, 2,4-, 2,5- oder 2,6-Dimethylcyclohexyl, Cycloheptyl oder Cyclooctyl;
- als C₇- bis C₁₈-Aralkyl-, insbesondere C₇- bis C₁₂-Aralkylgruppen kommen insbesondere alkylsubstituierte Phenylalkylgruppen in Frage, z.B. Benzyl, 2-, 3- oder 4-Methylbenzyl, 2-Phenylethyl, 3-Phenylpropyl, 4-Phenylbutyl, 2-, 3- oder 4-Ethylbenzyl, 3- oder 4-Isopropylbenzyl oder 3- oder 4-Butylbenzyl;
- als C₆- bis C₁₈-Arylgruppen eignen sich beispielsweise Phenyl, 2-, 3- oder 4-Bisphenyl, α- oder β-Naphthyl, 2-, 3-oder 4-Methylphenyl, 2-, 3- oder 4-Ethylphenyl, 3- oder 4-lsopropylphenyl, 3- oder 4-Butylphenyl oder 3- oder 4-(2'-Ethylhexyl)phenyl; bevorzugt werden C₆- bis C₁₄-Arylgruppen, insbesondere Phenyl und alkylsubstituiertes Phenyl;
- als C₃- bis C₁₈-Heteroarylgruppen kommen insbesondere fünf- oder sechsgliedrige C₃- bis C₁₂-Heteroarylgruppen mit ein oder zwei Heteroatomen aus der Gruppe Stickstoff, Sauerstoff und Schwefel in Frage, Beispiele hierfür sind: 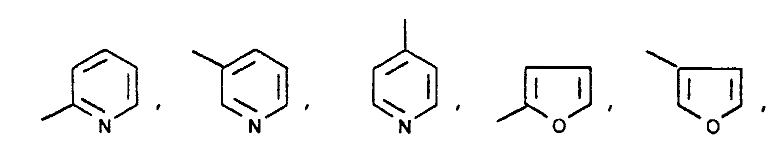 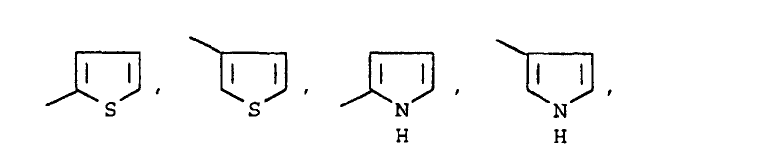 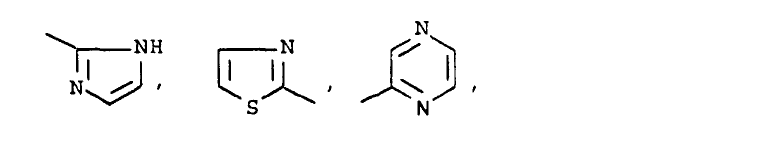 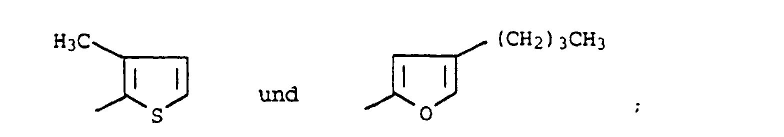
- als durch Sauerstoff oder Amino-Gruppierungen, insbesondere NH- oder N(CH₃)-Gruppierungen, unterbrochene aliphatische Reste kommen beispielsweise folgende Strukturen in Betracht:

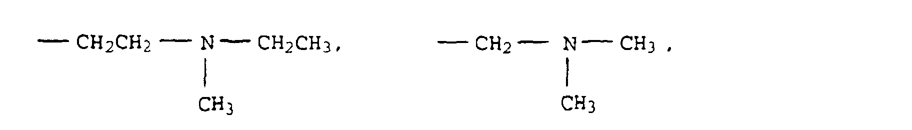

   ―CH₂―O―CH₃, ―CH₂CH₂―O―CH₃, ―CH₂CH₂―O―CH₂CH₃,

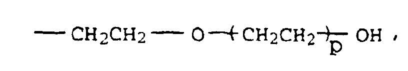

   ―(CH₂)₄―O―CH₃,

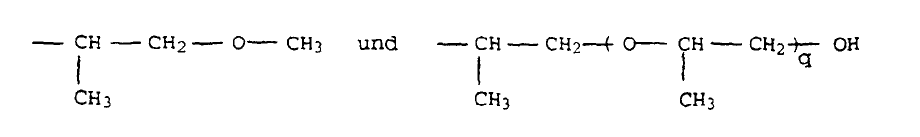

   mit p = 2 bis 8 und q = 2 bis 5.

Die Variable Z⁴ in den cyclischen Oxim-Gruppierungen L² kann vor allem C₃- bis C₁₂-Alkylengruppierungen der folgenden Struktur bedeuten:

―CH₂CH₂CH₂―,

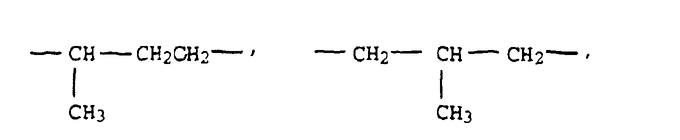

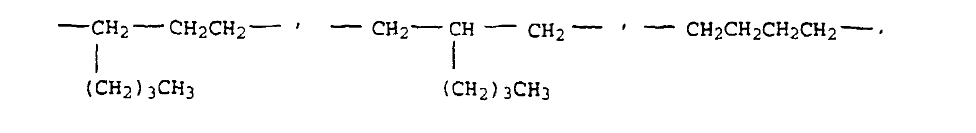

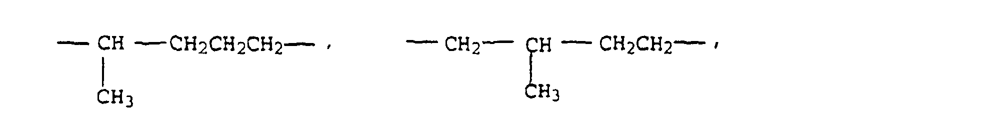

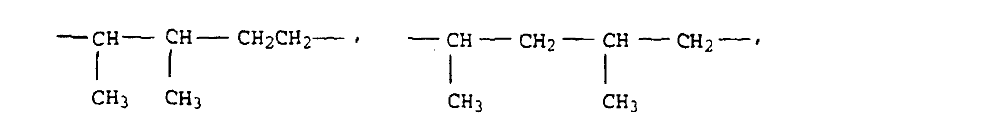

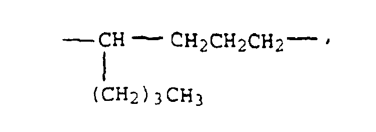

―CH₂CH₂CH₂CH₂CH₂―,

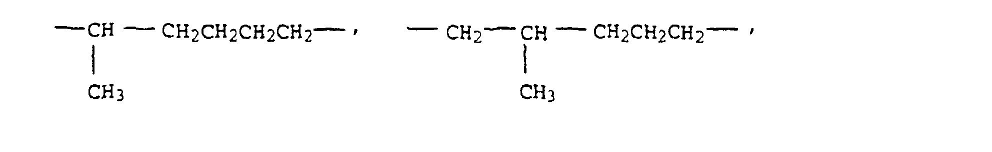

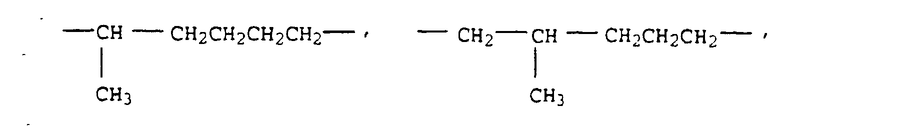

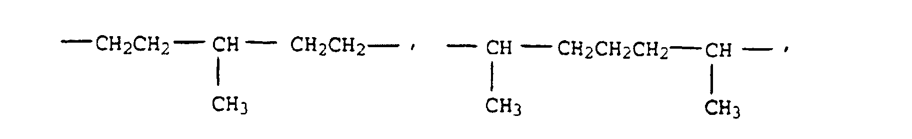

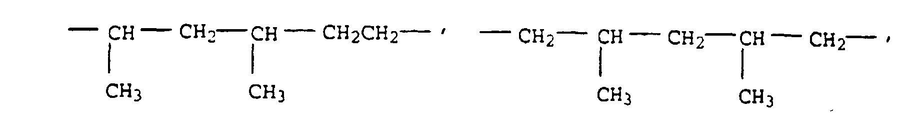

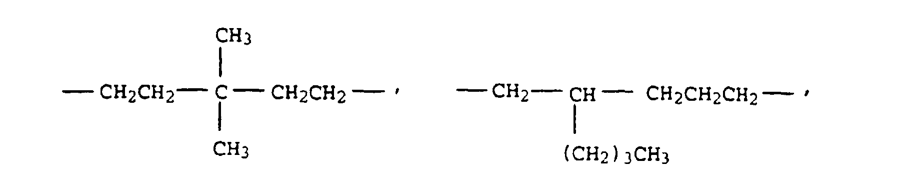

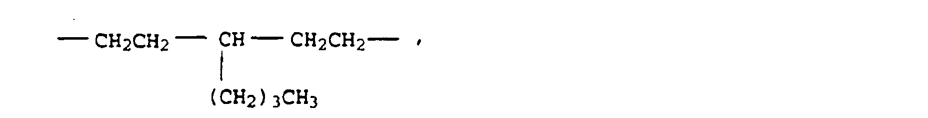

―(CH)₆―, ―(CH₂)₇― oder ―(CH₂)₈―,

wobei die Variable Z⁴ wie angegeben funktionalisiert oder unterbrochen sein kann.

Die Variablen Z¹ bis Z³ in den heterocyclischen Systemen (h), (j) und (k) können vor allem C₂- bis C₁₀-Alkylengruppierungen der folgenden Struktur bedeuten:

―CH₂CH₂―,

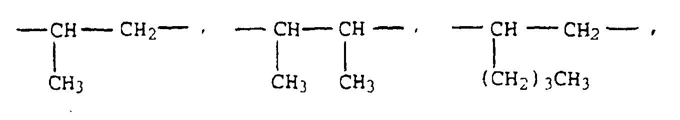

―CH₂CH₂CH₂―,

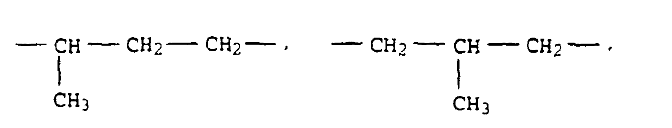

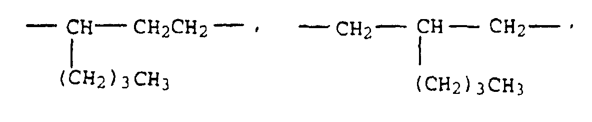

―CH₂CH₂CH₂CH₂―,

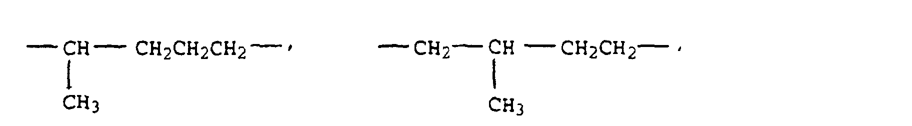

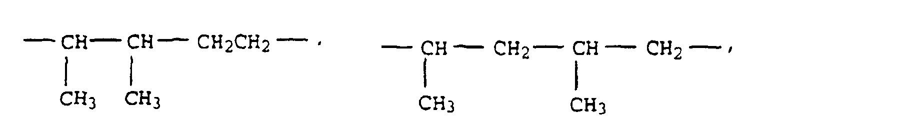

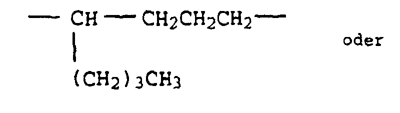

oder

―CH₂CH₂CH₂CH₂CH₂―,

wobei bei unsymmetrischen Alkylengruppierungen prinzipiell beide Einbaumöglichkeiten in die Ringe möglich sind. Die Variablen Z¹ bis Z³ können wie angegeben funktionalisiert oder unterbrochen sein.

Als gegebenenfalls substituierte Kohlenwasserstoffreste R¹ für die Variable L³ in den Oximestern II eignen sich vor allem C₁- bis C₁₈-Alkylreste, C₂- bis C₁₈-Alkenylreste, C₇- bis C₁₂-Aralkylreste oder Phenyl oder alkylsubstituiertes Phenyl mit insgesamt bis zu 14 C-Atomen. Typische Beispiele hierfür sind:

Methyl, Ethyl, n-Propyl, n-Butyl, n-Pentyl, n-Hexyl, n-Heptyl, n-Octyl, n-Nonyl, n-Decyl, n-Dodecyl, n-Tetradecyl, n-Hexadecyl, n-Octadecyl, Benzyl, 2-Phenylethyl, 4-Phenylbutyl, Phenyl und o-, m- oder p-Tolyl.

Als Carbonesterreste (a) für L³ eignen sich vor allem:

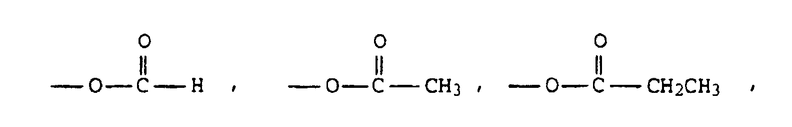

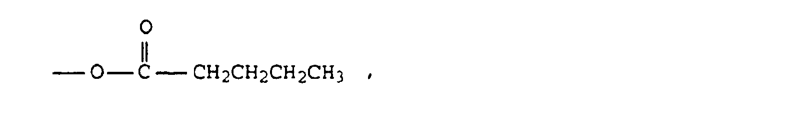

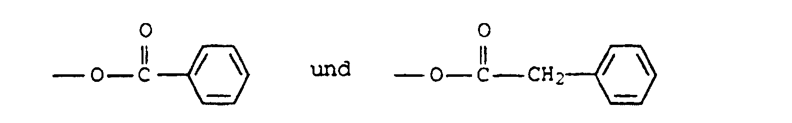

Als Carbonamidreste (b) für L³ eignen sich vor allem: 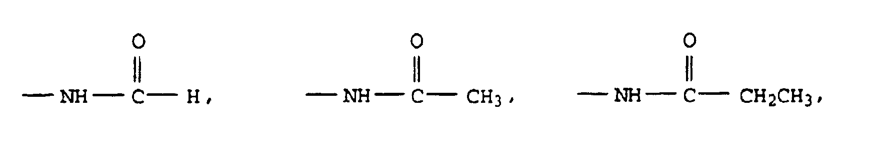 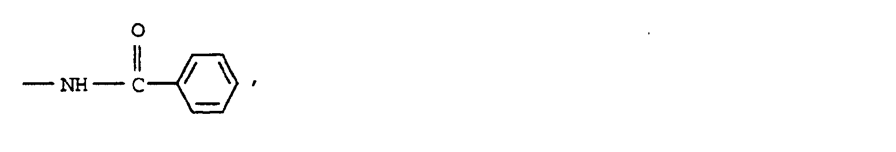 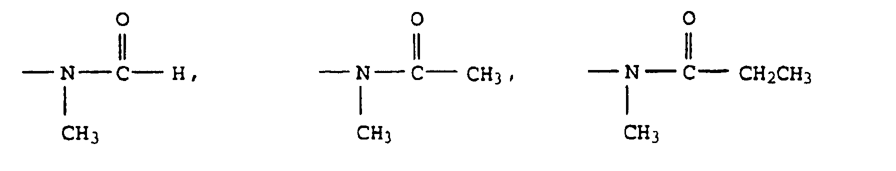 und

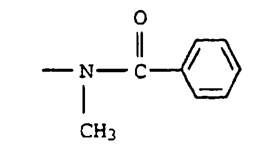

Als Phenolatreste (c) für L³ eignen sich vor allem: 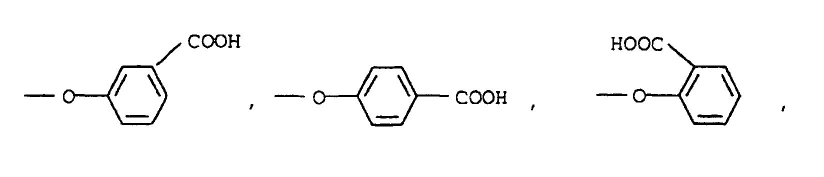 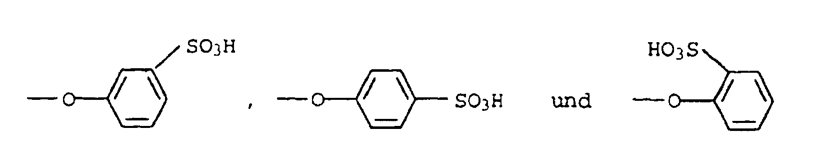 sowie die zugehörigen Natrium- oder Kaliumsalze.

Als Vinyloxyreste (d) für L³ eignen sich vor allem:

―O―CH=CH₂,

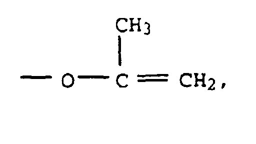

―O―CH=CH―CH₃,

―O―CH=CH―CH=CH₂,

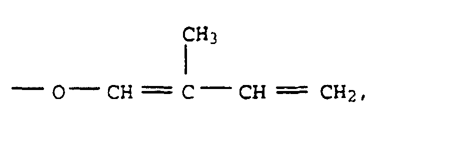

―O―CH=CH―CH=CH―CH₃,

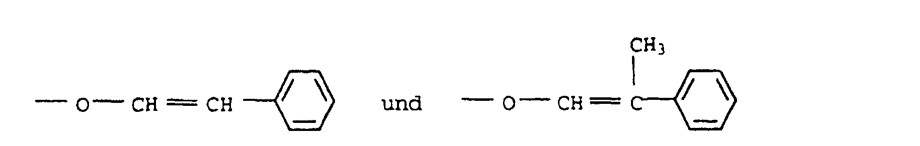

Als Sulfonamidreste (e) für L³ eignen sich vor allem:

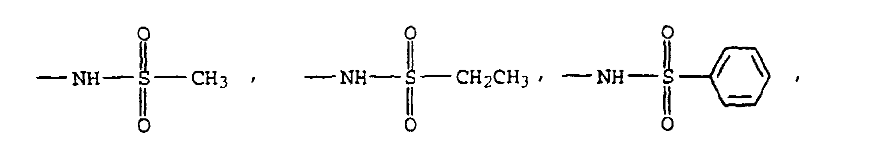

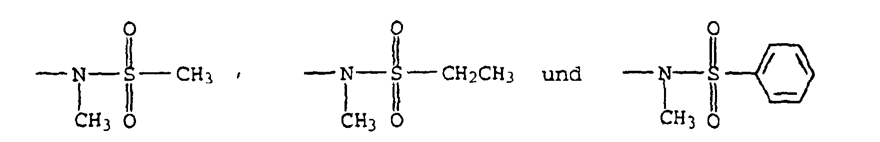

Als Imidazolreste (f) für L³ eignen sich vor allem:

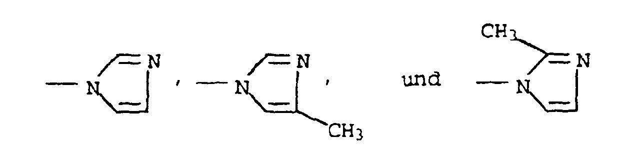

Als Amidolactamreste (g) für L³ eignen sich vor allem: 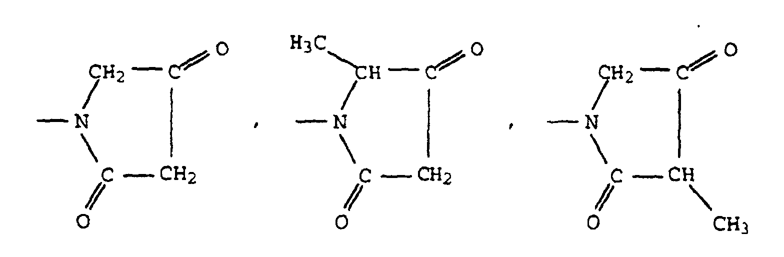 und

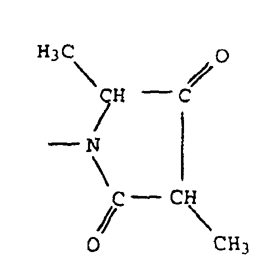

Als cyclische Carbamatreste (h) für L³ eignen sich vor allem:

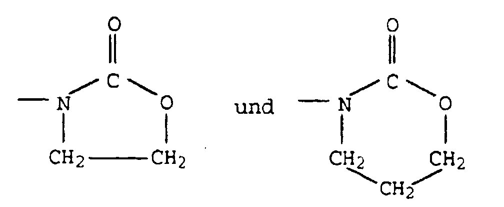

Als Lactonoxyreste (j) für L³ eignen sich vor allem:

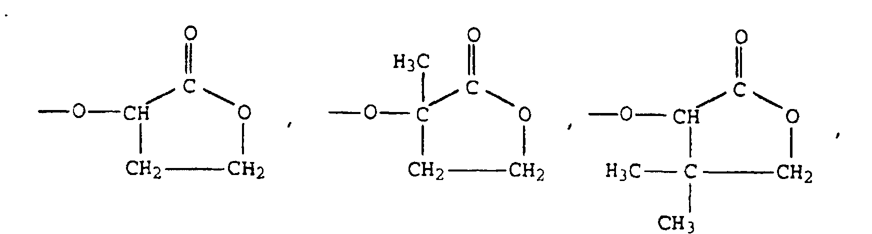

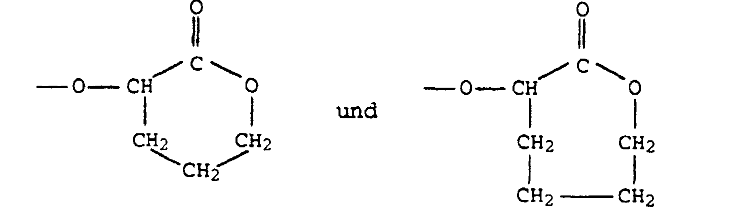

T in der allgemeinen Formel für den Lactonoxyrest (j) bedeutet vorzugsweise Wasserstoff oder Methyl.

Als Lactamreste (k) für L³ eignen sich vor allem:

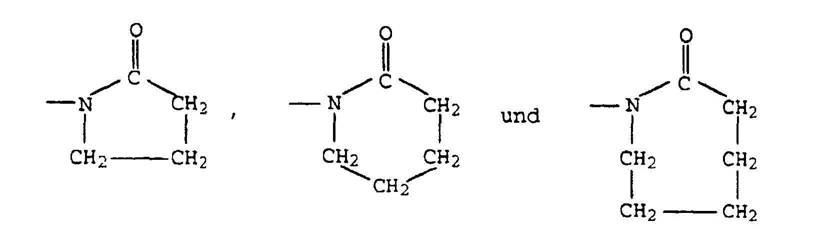

Typische Beispiele für das Brückenglied A in den Oximestern II sind ebenfalls die vorne für die sauerstoffhaltige Gruppe X in den heterocyclischen Verbindungen I angegebenen.

Das Brückenglied A in den Oximestern II steht insbesondere für eine chemische Bindung (formal abgeleitet von Oxalsäure) oder 1,2-Ethylen (abgeleitet von Bernsteinsäure), 1,4-Butylen (abgeleitet von Adipinsäure), Hexamethylen (abgeleitet von Korksäure), Octamethylen (abgeleitet von Sebacinsäure), 1,3-oder 1,4-Cyclohexylen oder 1,2-, 1,3- oder 1,4-Phenylen (abgeleitet von Phthalsäure, Isophthalsäure bzw. Terephthalsäure).

Die oben definierten Variablen R¹, R², R³, Z¹ bis Z⁴ und A können zusätzlich durch die angegebenen Gruppen funktionalisiert sein. Dabei bedeuten C₁- bis C₄-Alkoxygruppen insbesondere Methoxy, Ethoxy, n-Propoxy, iso-Propoxy, n-Butoxy, iso-Butoxy, sec.-Butoxy oder tert.-Butoxy. Als Amino-Gruppierungen werden -NH₂, -NH(CH₃), -NH (CH₂CH₃), -N(CH₃)₂ und -N(CH₂CH₃)₂ bevorzugt. Carboxy-C₁- bis C₄-alkylgruppen sind beispielsweise Carboxymethyl, Carboxyethyl, Carboxypropyl, Carboxybutyl oder Carboxy-tert.-butyl.

In einer bevorzugten Ausführungsform werden Oximester II eingesetzt, bei denen L² für eine Oxim-Gruppierung der Formel 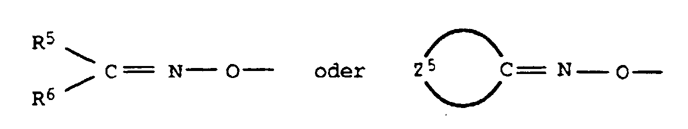 steht, wobei
- R⁵ und R⁶: Wasserstoff, C₁- bis C₄-Alkyl, insbesondere Methyl oder Ethyl, Phenyl oder Benzyl bedeutet und
- Z⁵: 1,4-Butylen, 1,5-Pentylen oder 1,6-Hexylen bezeichnet.

Derartige Aldoxim- oder Ketoxim-Gruppierungen leiten sich von gängigen Aldehyden oder Ketonen ab, beispielsweise von Formaldehyd, Acetaldehyd, Propionaldehyd, Benzaldehyd, Phenylacetaldehyd, Aceton, Ethylmethylketon, Diethylketon, Acetophenon, Phenylaceton, Benzophenon, Cyclopentanon, Cyclohexanon oder Cycloheptanon.

Weiterhin werden Oximester II bevorzugt, bei denen L³ für eine zweite Oxim-Gruppierung L² steht; besonders bevorzugt werden hierbei solche Oximester II, bei denen L² und L³ für die gleiche Oxim-Gruppierung stehen.

Von besonderem Interesse sind Bisoximester II, die sich von Oxalsäure, Bernsteinsäure, Adipinsäure, Phthalsäure, Isophthalsäure oder Terephthalsäure und aliphatischen Ketonen mit 3 bis 6 C-Atomen oder von C₅- bis C₇-Cycloalkanonen ableiten. Derartige Systeme lassen sich in einfacher Weise beispielsweise durch Umsetzung der entsprechenden Dicarbonsäurechlorideoder-bromide mit den entsprechenden aliphatischen bzw. cycloaliphatischen Ketoximen in Gegenwart von Basen herstellen.

Von besonderem Interesse sind weiterhin auch als Bisoximester II Bisiminocarbonate, die sich formal von der Kohlensäure und aliphatischen Ketonen mit 3 bis 6 C-Atomen oder von C₅- bis C₇-Cycloalkanonen ableiten. Derartige Systeme lassen sich in einfacher Weise beispielsweise durch Umsetzung von Phosgen mit den entsprechenden aliphatischen bzw. cycloaliphatischen Ketoximen in Gegenwart von Basen herstellen.

Die beschriebenen Oximester II, Methoden für ihre Herstellung und ihre Verwendung als Bleichaktivatoren in Wasch-, Bleich- und Reinigungsmitteln sind im Prinzip bekannt, beispielsweise aus der JP-A 06/336 468 (5), der WO-A 93/04037 (6), dem Zeitschriftenartikel von A. Jumar, P.Held und W. Schulze in Z. Chem. 7 (1967), S. 344 - 345 (7) oder der deutschen Patentanmeldung 1 95 41 012.2 (8). Gemäß (7) lassen sich unsymmetrische Bisoximester II und Monoximester II für den Fall m = 0 (Kohlen säurederivate) über die Zwischenstufe des entsprechenden Chlorformyloxims herstellen.

Selbstverständlich können auch Mischungen der heterocyclischen Verbindungen I mit den Oximestern II in den erfindungsgemäßen festen Zusammensetzungen eingesetzt werden.

Eine hohe innere Oberfläche der inerten porösen Trägermaterialien ist von ausschlaggebender Bedeutung für die gewünschten Eigenschaften der resultierenden festen Zusammensetzung. Die innere Oberfläche liegt vorzugsweise im Bereich von 50 bis 480 m²/g, insbesondere 100 bis 460 m²/g, vor allem 180 bis 450 m²/g.

Eine Bedeutung hat auch die durchschnittliche Teilchengröße der inerten porösen Trägermaterialien, sie kann jedoch in einem größeren Bereich schwanken als die innere Oberfläche. Die besten Ergebnisse erzielt man mit einer durchschnittlichen Teilchengröße von 10 nm bis 100 µm, insbesondere 20 nm bis 50 µm, vor allem 1 µm bis 20 µm, es können jedoch - je nach Art des Trägermaterials und Anwendung der erfindungsgemäßen festen Zusammensetzung - auch kleinere durchschnittliche Teilchengrößen, in etwa herunter bis zu 3 nm, oder größere, in etwa hinauf bis zu 2 mm, insbesondere bis zu 500 µm, vorliegen.

Als inerte poröse Trägermaterialien können im Prinzip alle üblichen Arten von derartigen chemisch inerten Materialien verwendet werden. Insbesondere eignen sich jedoch Kieselgele, Kieselsäuren, Aluminiumoxide, Kaoline oder Aluminiumsilicate oder Mischungen hieraus.

Kieselgele (Silicagele, Kieselsäuregele) sind kolloidale geformte oder ungeformte Kieselsäuren von elastischer bis fester Konsistenz mit lockerer bis dichter Porenstruktur und hohem Adsorptionsvermögen. Kieselgel-Oberflächen weisen Aciditätseigenschaften auf. Kieselgel stellt man üblicherweise aus Wasserglas durch Umsetzung mit Mineralsäuren her.

Unter den Kieselsäuren lassen sich neben den im Naßverfahren hergestellten Kieselsäuren besonders vorteilhaft die thermisch gewonnenen, d.h. üblicherweise durch Flammenhydrolyse von SiCl₄ hergestellten hochdispersen "pyrogenen" SiO₂-Qualitäten einsetzen (z.B. Aerosile® oder Sipernate®). In einer besonders bevorzugten Ausführungsform der vorliegenden Erfindung wird Kieselsäure mit einer durchschnittlichen (Agglomerat-)Teilchengröße von 100 nm bis 30 µm, insbesondere 1 µm bis 20 µm, und einem SiO₂-Gehalt von 95 bis 100, vorzugsweise 98 bis 100 Gew.-%, verwendet.

Aluminiumoxide kommen in der Natur beispielsweise als Tonerde oder als Korund vor. Hierbei liegt das Aluminiumoxid in der α-Modifikation vor. Technisch wird α-Al₂O₃ aus Bauxit nach dem Bayer-Verfahren gewonnen. Als Adsorbientien besonders geeignete "aktive" Aluminiumoxide mit hoher spezifischer Oberfläche werden durch Fällungsverfahren aus Aluminium-Salzlösungen oder durch Calcination aus α-Aluminiumhydroxid hergestellt.

Kaoline sind im Boden natürlich vorkommende hydratisierte Aluminiumsilicate (Tone), die wegen ihrer früheren Hauptverwendung auch Porzellanerden ("China-Clays") genannt werden. Hauptbestandteile sind der trikline Kaolinit und die monoklinen Dickit und Nakrit zusammen mit Montmorillonit und gelförmigen Tonerdesilicaten (Allophanen).

Aluminiumsilicate sind Verbindungen mit unterschiedlichen Anteilen Al₂O₃ und SiO₂, die in der Natur als Andalusit, Disthen, Mullit, Sillimanit usw. vorkommen. Aluminiumsilicat-Minerale, in denen Al anstelle von Si Gitterplätze im Kristallgitter einnimmt, sind die Alumosilicate (z.B. Ultramarine, Zeolithe, Feldspäte).

Frisch gefällte Aluminiumsilicate sind feindispers und weisen eine große Oberfläche und hohes Adsorptionsvermögen auf.

Das Verhältnis von heterocyclischen Verbindungen I bzw. Oximestern II und inerten porösen Trägermaterialien in der erfindungsgemäßen festen Zusammensetzung kann innerhalb gewisser Grenzen schwanken, je nach Herstellungsmethode der festen Zusammensetzung und Stoffeigenschaften der eingesetzten Komponenten. Ein bevorzugtes Verhältnis ist 10 bis 95 Gew.-Teile I bzw. II zu 5 bis 90 Gew.-Teilen Träger, insbesondere 30 bis 90 Gew.-Teile I bzw. II zu 10 bis 70 Gew.-Teilen Träger. Die angegebenen Gew.-Teile I bzw. II beziehen sich immer auf wasserfreie bzw. lösungsmittelfreie Verbindung I bzw. II. Aus Gründen der Wirtschaftlichkeit wird ein möglichst hoher Anteil an heterocyclischen Verbindungen I bzw. Oximestern II angestrebt.

Die erfindungsgemäße feste Zusammensetzung ist homogen aus den eingesetzten Komponenten aufgebaut. Die heterocyclischen Verbindungen I bzw. die Oximester II und gegebenenfalls weitere Hilfsstoffe ziehen in die inneren Bereiche der Trägermaterial-Körner gleichmäßig ein und verteilen sich dort gleichmäßig, da diese eine relativ hohe innere Oberfläche aufweisen. Normalerweise liegt kein merkliches Konzentrationsgefälle der einziehenden Substanzen zwischen Innerem und äußerer Oberfläche der Körner oder Körner-Aggregate vor, so daß man nicht von "Beschichtung" oder "Coating" sprechen kann.

Es kann für die Eigenschaften der erfindungsgemäßen festen Zusammensetzung vorteilhaft sein, wenn diese zusätzlich zu den angegebenen Gew.-Teilen an I bzw. II und Trägermaterial 0,5 bis 70 Gew.-Teile, insbesondere 2 bis 40 Gew.-Teile, vor allem 5 bis 25 Gew.-Teile, anionischer, nichtionischer oder zwitterionischer Tenside, carboxylgruppenhaltiger Polymerisate, Polysaccharide, Polyalkylenglykole, saurer Alkalimetall- oder Erdalkalimetallsalze anorganischer Säuren, neutraler Alkalimetallsalze, aliphatischer C₈- bis C₁₈-Monocarbonsäuren, aliphatischer Di- oder Tricarbonsäuren, aromatischer Mono- oder Dicarbonsäuren, wobei die vorgenannten Carbonsäuren zusätzlich Hydroxylund Aminogruppen im Molekül enthalten können, aliphatischer C₃- bis C₇-Monohydroxycarbonsäuren oder von Mischungen hieraus enthalten.

Als anionische Tenside kommen hier z.B. Alkalimetallsalze von Fettsäuren (Seifen), Alkylbenzolsulfonate, Alkansulfonate, α-Olefinsulfonate, Hydroxyalkansulfonate, α-Sulfofettsäureester, Alkylsulfate, Alkylethersulfate oder Fettalkoholethersulfate in Betracht. Bevorzugt werden hiervon Alkylbenzolsulfonate, insbesondere die Alkalimetallund Ammoniumsalze von linearen C₁₁- bis C₁₃-Alkylbenzolsulfonaten, z.B. Natriumdodecylbenzolsulfonat.

Als nichtionische Tenside dienen hier beispielsweise Alkoholoxalkylate, insbesondere Fettalkoholoxalkylate, sowie Alkylphenoloxalkylate, Fettsäurealkylolamide und Alkylglykoside, insbesondere C₈- bis C₁₆-Monoalkylglucoside. Bevorzugt werden hiervon Ethoxylate und Propoxylate von gesättigten oder ungesättigten C₁₂- bis C₂₀-Fettalkoholen, z.B. Kokosfett- und Talgfettalkoholethoxylate.

Als zwitterionische Tenside kommen hier beispielsweise sekundäre und tertiäre Aminoxide in Frage.

Als carboxylgruppenhaltige Polymerisate kommen vor allem Homopolymerisate der Acrylsäure und Methacrylsäure sowie Acrylsäure und/oder Methacrylsäure enthaltende Copolymerisate sowie die zugehörigen Alkalimetall- oder Ammoniumsalze in Betracht. Besonders bevorzugt werden Acrylsäure-Maleinsäure-Copolymerisate, insbesondere in teilneutralisierter Form, z.B. zu ca. 50 % als Natriumsalz vorliegendes Acrylsäure-Maleinsäure-Copolymerisat (Gewichtsverhältnis Acrylsäure : Maleinsäure = 70 : 30).

Als Polysaccharide dienen am vorteilhaftesten Stärke, Amylose sowie Derivate dieser natürlich vorkommenden Polysaccharide wie Carboxymethylcellulose, Celluloseacetathydrogenphthalat, Ethylcellulose oder sulfatierte Celluloseether. Am günstigsten kann hiervon Stärke selbst eingesetzt werden.

Als Polyalkylenglykole eignen sich vor allem Polyethylenglykole und Ethylenoxid-Propylenoxid-Copolymere.

Als saure Alkalimetall- und Erdalkalimetallsalze anorganischer Säuren kommen vor allem Natriumhydrogencarbonat und Natriumhydrogensulfat, daneben aber auch Kaliumhydrogencarbonat, Kaliumhydrogensulfat, Natriumdihydrogenphosphat, Kaliumdihydrogenphosphat, Dinatriumhydrogenphosphat, Dikaliumhydrogenphosphat, Calciumhydrogenphosphat oder Magnesiumhydrogenphosphat in Betracht.

Als neutrale Alkalimetallsalze eignen sich insbesondere Natriumsulfat und Natriumchlorid.

Als aliphatische C₈- bis C₁₈-Monocarbonsäuren, aliphatische Di- oder Tricarbonsäuren, aromatische Monooder Dicarbonsäuren, wobei vorgenannte Carbonsäuren zusätzlich Hydroxyl- und Aminogruppen im Molekül enthalten können, und als aliphatische C₃- bis C₇-Monohydroxycarbonsäuren eignen sich z. B. Fettsäuren wie Laurinsäure, Myristinsäure, Palmitinsäure und Stearinsäure, Oxalsäure, Malonsäure, Bernsteinsäure, Glutarsäure, Adipinsäure, Pimelinsäure, Korksäure, Maleinsäure, Fumarsäure, Weinsäure, Äpfelsäure, Citronensäure, Benzoesäure, Salicylsäure, Anthranilsäure, Sulfanilsäure, Phthalsäure, α- und β-Naphthoesäure, Naphthalsäure und Milchsäure. Bevorzugt werden hiervon Palmitinsäure, Stearinsäure, Oxalsäure, Malonsäure, Bernsteinsäure, Glutarsäure, Adipinsäure, Pimelinsäure, Weinsäure, Citronensäure und Benzoesäure. Die genannten Carbonsäuren bewirken hauptsächlich eine Verbesserung der Farbstabilität bei der Lagerung in Waschpulvern.

Es können auch Mischungen aus mehreren der genannte Zusatzstoffen aus einer der aufgezählten Gruppen oder aus verschiedenen Gruppen eingesetzt werden.

Gegenstand der Erfindung ist auch ein Verfahren zur Herstellung der beschriebenen festen Zusammensetzung, wie in den Ansprüchen definiert.

In einer bevorzugten Ausführungsform wird dieses Herstellungsverfahren so durchgeführt, daß man eine Lösung oder eine Schmelze der heterocyclischen Verbindungen I und/oder der Oximester II durch Einrühren, Aufsprühen oder Tränken mit den inerten porösen Trägermaterialien mischt, das Lösungsmittel, sofern vorhanden, durch Destillation oder Trocknen weitgehend entfernt und, wenn notwendig, die resultierende Mischung, gegebenenfalls nach Vermischen mit weiteren Hilfsstoffen, durch übliche Methoden in eine für den jeweiligen Anwendungszweck geeignete konfektionierte Form bringt. Als Lösungsmittel eignen sich hierbei vor allem übliche organische Lösungsmittel wie Alkohole, z.B. Methanol, Ethanol oder Isopropanol, Ketone, z.B. Aceton, Carbonsäureester, z.B. Essigsäuremethyl oder -ethylester, oder gegebenenfalls halogenierte Kohlenwasserstoffe, z.B. n-Hexan, Cyclohexan, Toluol, Xylol oder Chlorbenzol.

Ein typisches derartiges Herstellungsverfahren sieht so aus, daß man die inerten porösen Trägermaterialien in die Lösung der Verbindungen I bzw. II in einem organischen Lösungsmittel einträgt und gegebenenfalls mit weiteren Hilfsstoffen, insbesondere Tensiden, untermischt. Anschließend wird das Lösungsmittel praktisch vollständig entfernt, dies geschieht vorteilhafterweise temperaturschonend durch Destillation bei vermindertem Druck, insbesondere wenn ein relativ hochsiedendes Lösungsmittel eingesetzt wurde. Danach, wenn notwendig, wird das Produkt, welches als rieselfähiges Pulver anfällt, nach üblichen Methoden kompaktiert, gegebenenfalls zerkleinert und auf eine Größenfraktion im Bereich von beispielsweise 200 µm bis 3 mm - je nach Anwendung - gesiebt.

Falls erforderlich kann die Lagerstabilität der erfindungsgemäßen festen Zusammensetzung durch weitere Nachbehandlungen verbessert werden, beispielsweise durch Beschichten mit Fetten, Ölen, Schmelzen oder Lösungen.

Anstelle des Eintragens und Untermischens (Einrührens) der Trägermaterialien kann die Lösung der Verbindungen I bzw. II auch auf die Trägermaterialien nach üblichen Techniken aufgesprüht werden. Auch Tränkverfahren sind anwendbar. Sind nach dem Aufsprüh- oder Tränkverfahren bereits pulvrige Mischungen vorhanden, können diese auch direkt ohne weitere Trocknung in einem üblichen Mischer agglomeriert werden, gegebenenfalls müssen weitere Hilfsstoffe, insbesondere Tenside, beispielsweise in geschmolzener Form oder als hochkonzentrierte wäßrige Lösung, zugegeben werden, danach schließt sich gegebenenfalls die oben beschriebene Konfektionierung auf die gewünschte Größenfraktion an.

Die erfindungsgemäße feste Zusammensetzung eignet sich hauptsächlich als festes Additiv für Wasch-, Bleich- und Reinigungsmittel, insbesondere als stabile Bleichaktivator-Komponente in derartigen Mitteln.

So lassen sich Wasch-, Bleich- und Reinigungsmittel formulieren, die 0,1 bis 30 Gew.-%, bezogen auf die Gesamtmenge der Formulierung, der erfindungsgemäßen festen Zusammensetzung enthalten. Diese Wasch-, Bleichund Reinigungsmittel sind ebenfalls Gegenstand der vorliegenden Erfindung. Die weiteren üblichen Komponenten für Wasch-, Bleich- und Reinigungsmittel werden im folgenden näher erläutert:

Die beschriebenen Kaltbleichaktivatoren I bzw. II bilden zusammen mit Bleichmitteln, in der Regel Perverbindungen, welche ebenfalls normalerweise separat in der Formulierung vorliegen, das Bleichsystem. Dabei kann der pH-Wert der Wasch- bzw. Bleich- bzw. Reinigungsflotte in weiten Grenzen, vom schwach sauren Bereich (pH 4) bis in den stark alkalischen Bereich (pH 13), je nach Anwendungszweck, gewählt werden. Bevorzugt wird der alkalische Bereich von pH 8 bis pH 11, da erfür die Aktivierungsreaktion und die Stabilität der gebildeten Perverbindung besonders vorteilhaft ist.

Aus diesem Grunde werden die beschriebenen Bleichaktivatoren auch bevorzugt zusammen mit einem Natriumperborat oder mit Natriumcarbonat-Perhydrat verwendet, die in ihren Lösungen bereits pH-Werte dieses Bereichs aufweisen. Beispiele anderer geeigneter Perverbindungen sind Phosphat-Perhydrate und Harnstoff-Perhydrat. Gelegentlich kann es auch zweckmäßig sein, den pH-Wert des Mediums nach erfolgter Aktivierungsreaktion durch geeignete Zusätze nochmals, vor allem in den sauren Bereich, zu verschieben.

Die Einsatzmengen an Bleichmitteln (Perverbindungen) werden im allgemeinen so gewählt, daß in den Flotten zwischen 10 und 10000 ppm Aktivsauerstoff, vorzugsweise zwischen 50 und 5000 ppm Aktivsauerstoff, vorhanden sind. Auch die verwendete Menge an Bleichaktivator hängt vom Anwendungszweck ab. Je nach gewünschtem Aktivierungsgrad werden 0,03 bis 1,0 Mol, vorzugsweise 0,1 bis 0,5 Mol Aktivator pro Mol anorganischer Perverbindung verwendet, doch können in besonderen Fällen diese Grenzen auch über- oder unterschritten werden.

Als zusätzliche Bleichaktivatoren, die in Kombination mit den heterocyclischen Verbindungen I bzw. den Oximestern II (in der erfindungsgemäßen festen Zusammensetzung oder als gegebenenfalls konfektionierte separate Komponente) eingesetzt werden können, kommen vor allem in Betracht:
- polyacylierte Zucker, z.B. Pentaacetylglucose;
- Acyloxybenzolsulfonsäuren und deren Alkali- und Erdalkalimetallsalze, z.B. Natrium-p-isononanoyloxy-benzolsulfonat oder Natrium-p-benzoyloxy-benzolsulfonat;
- N,N-diacylierte und N,N,N',N'-tetraacylierfe Amine, z.B. N,N,N',N'-Tetraacetyl-methylendiamin und -ethylendiamin, N,N-Diacetylanilin, N,N-Diacetyl-p-toluidin oder 1,3-diacylierte Hydantoine wie I,3-Diacetyl-5,5-dimethylhydantoin;
- N-Alkyl-N-sulfonyl-carbonamide, z.B. N-Methyl-N-mesyl-acetamid oder N-Methyl-N-mesyl-benzamid;
- N-acylierte cyclische Hydrazide, acylierte Triazole oder Urazole, z.B. Monoacetyl-maleinsäurehydrazid;
- O,N,N-trisubstituierte Hydroxylamine, z.B. O-Benzoyl-N,N-succinylhydroxylamin, O-Acetyl-N,N-succinyl-hydroxylamin oder O,N,N-Triacetylhydroxylamin;
- N,N'-Diacyl-sulfurylamide, z.B. N,N'-Dimethyl-N, N'-diacetylsulfurylamid oder N,N'-Diethyl-N,N'-dipropionyl-sulfurylamid;
- Triacylcyanurate, z.B. Triacetylcyanurat oder Tribenzoylcyanurat;
- Carbonsäureanhydride, z.B. Benzoesäureanhydrid, m-Chlorbenzoesäureanhydrid oder Phthalsäureanhydrid;
- 1,3-Diacyl-4,5-diacyloxy-imidazoline, z.B. 1,3-Diacetyl-4,5-diacetoxyimidazolin;
- Tetraacetylglycoluril und Tetrapropionylglycoluril;
- diacylierte 2,5-Diketopiperazine, z.B. 1,4-Diacetyl-2,5-diketopiperazin;
- Acylierungsprodukte von Propylendiharnstoff und 22-Dimethylpropylendihamstoff, z.B. Tetraacetylpropylendiharnstoff;
- α-Acyloxy-polyacyl-malonamide, z.B. α-Acetoxy-N,N'-diacetylmalonamid;
- Diacyl-dioxohexahydro-1,3,5-triazine, z.B. 1,5-Diacetyl-2,4-dioxohexahydro-1,3,5-triazin;
- Benz-(4H)1,3-oxazin-4-one mit Alkylresten, z.B. Methyl, oder aromatischen Resten, z.B. Phenyl, in der 2-Position.

Auf dem Gebiet der Textilwäsche können die beschriebenen Bleichaktivatoren mit nahezu allen üblichen Bestandteilen von Waschmittelformulierungen kombiniert werden. Man kann auf diese Weise Formulierungen aufbauen, die sich speziell zur Textilbehandlung bei niedrigen Temperaturen eignen, und auch solche, die in mehreren Temperaturbereichen bis hinauf zum traditionellen Bereich der Kochwäsche geeignet sind.

Hauptbestandteile solcher Waschmittelformulierungen sind, neben Bleichmitteln (Perverbindungen) und Bleichaktivatoren, Gerüstsubstanzen (Builder) und Tenside. Daneben können andere übliche Hilfsstoffe und Begleitstoffe wie Vergrauungsinhibitoren, Peroxidstabilisatoren, Elektrolyte, optische Aufheller, Enzyme, Parfümöle, Schaumregulatoren und aktivierende Substanzen in diesen Mitteln vorliegen, wenn dies zweckmäßig ist. Die Komponenten für Bleich- und Reinigungsmittelformulierungen sind im Prinzip die gleichen.

Beispiele üblicher Gerüstsubstanzen sind kondensierte Phosphate, Alkalisilicate, Alkalicarbonate, Salze von Aminocarbonsäuren wie Nitrilotriessigsäure, Salze von Polyphosphonsäuren wie Hydroxyethandiphosphonsäure, Salze von Polycarbonsäuren wie Citronensäure oder Polyacrylsäure und unlösliche Natriumaluminiumsilicate vom Typ Zeolith NaA und NaX.

Als Tenside kommen insbesondere solche vom Typ der nichtionischen und synthetischen anionischen Tenside in Frage. Beispiele für nichtionische Tenside sind die aus langkettigen Alkoholen oder Alkylphenolen und Ethylenoxid hergestellten Polyethylenglykolmonoalkyl- und Polyethylenglykolmonophenylether sowie die langkettigen Alkylglycoside.

Bei den anionischen Tensiden handelt es sich in erster Linie um Sulfate und Sulfonate langkettiger Verbindungen, beispielsweise Alkylbenzolsulfonate, Fettsäureestersulfonate, Alkansulfonate, Olefinsulfonate, Fettalkoholsulfate und Sulfate von Polyethylenglykolmonoethern. Weiterhin können Seifen und Salze langkettiger Acylcyanamide sowie langkettige Succinate und Sulfosuccinate verwendet werden.

Typische derartige Textilwasch- und -bleichmittel haben etwa die folgende Zusammensetzung:
0,5 bis 30 Gew.-%, vorzugsweise 5 bis 25 Gew.-% anionische und/oder nichtionische Tenside,
0,5 bis 60 Gew.-%, vorzugsweise 5 bis 50 Gew.-% Gerüstsubstanzen aus der Gruppe kondensierte Phosphate, Alkalisilicate, Alkalicarbonate, Natriumaluminiumsilicate und deren Mischungen,
0 bis 20 Gew.-%, vorzugsweise 0,5 bis 8 Gew.-% Gerüstsubstanzen aus der Gruppe Salze von Aminocarbonsäuren, Salze von Polyphosphonsäuren, Salze von Polycarbonsäuren und deren Mischungen,
2 bis 35 Gew.-%, vorzugsweise 5 bis 30 Gew.-% anorganische Perverbindungen,
0,1 bis 30 Gew.-%, vorzugsweise 0,5 bis 20 Gew.-% der erfindungsgemäßen festen Zusammensetzung,
ad 100 % übliche Hilfs- und Begleitstoffe und Wasser.

Die erfindungsgemäße feste Zusammensetzung weist den Vorteil auf, daß sie ausreichend lange lagerstabil ist, d. h. rieselfähig bleibt, ohne zu verklumpen oder zu verbacken. Während dieser Lagerzeit treten keine merklichen Aktivitätsverluste, beispielsweise hinsichtlich der Bleichwirkung, auf. Wasch-, Bleich- und Reinigungsmittel, welche die erfindungsgemäße feste Zusammensetzung enthalten, sind meist besser wirksam als solche, die übliche Bleichaktivatoren wie N,N,N',N'-Tetraacethylethylendiamin (TAED) in den gleichen Mengen enthalten.

### Herstellungsbeispiele

### Beispiel 1

10 g N-(n-Octanoyl)-ε-caprolactam wurden in 20 g Aceton gelöst und 10 g hochdisperse Kieselsäure mit einer inneren Oberfläche von ca. 190 m²/g und einer durchschnittlichen Teilchengröße von ca. 7 µm (Sipernat® 22S der Fa. Degussa) wurden zugegeben. Nach Durchmischen der Suspension wurde das Lösungsmittel destillativ entfernt. Zurück blieb ein trockenes, rieselfähiges Pulver, welches den Kaltbleichaktivator gleichmäßig verteilt enthielt.

### Beispiel 2

10 g N-(n-Octanoyl)-ε-caprolactam wurden in 20 g Aceton gelöst und 10 g der gleichen hochdispersen Kieselsäure wie in Beispiel 1, 2 Natriumsulfat, 2 g Citronensäure und 1 g Maisstärke wurden zugegeben. Nach Durchmischen der Suspension wurde das Lösungsmittel destillativ entfernt. Zurück blieb ein trockenes, rieselfähiges Pulver, welches den Kaltbleichaktivator gleichmäßig verteilt enthielt.

### Anwendungstechnische Untersuchungen

Mit dem Produkt aus Beispiel 2 bzw. zum Vergleich mit nicht konfektioniertem N-(n-Octanoyl)-e-caprolactam und N,N,N',N'-Tetraacetylethylendiamin (TAED) wurden Waschversuche mit hydrophilen und hydrophoben Testanschmutzungen durchgeführt. Dabei zeigte sich, daß der erfindungsgemäß konfektionierte Bleichaktivator besonders bei hydrophoben Anschmutzungen wie Gras auf Baumwolle eine deutlich höhere Bleichwirkung aufweist als der den Stand der Technik darstellende Bleichaktivator TAED (s. Tabelle 1). Die Prüfung erfolgte im Launder-o-meter, Typ Atlas Standard, unter Verwendung eines Vollwaschmittels der folgenden Zusammensetzung (Gew.-%):

| | |
|---|---|
| lineares Alkylbenzolsulfonat (50 gew.-%ig) | 0,8 |
| übliche Seife | 0,4 |
| übliches Fettalkoholsulfat | 12,0 |
| C₁₃/C₁₅-Oxoalkohol, mit 7 mol Ethylenoxid umgesetzt | 4,7 |
| Magnesiumsilikat | 0,8 |
| Natriumhydrogencarbonat | 9,0 |
| Natriumpercarbonat | 18,0 |
| Natriumsulfat | 3,2 |
| Natriumcitrat-Dihydrat | 5,0 |
| übliches Schichtsilikat | 14,0 |
| Zeolith A | 15,0 |
| Carboxymethylcellulose | 0,6 |
| Wasser | Rest zu 100 |

Die Einsatzkonzentration des Bleichaktivators gemäß Tabelle 1 oder 2 betrug jeweils 5 Gew.-% Aktivsubstanz, bezogen auf die Waschmittelmenge.

Die Versuchsdurchführung erfolgte unter folgenden Bedingungen:

| | |
|---|---|
| Flottenmenge | 250 g |
| Wasserhärte | 3,0 mmol/l |
| Ca/Mg/HCO₃-Molverhältnis | 4:1:8 |
| Waschmitteldosierung | 4,5 g/l |
| Temperatur | 22°C/38°C/60°C |
| Waschdauer | 30 min |
| Schmutzgewebe 1 | 2,5 g mit Chlorophyll angeschmutzte gebleichte Baumwolle (WFK Krefeld) |
| Schmutzgewebe 2 | 2,5 g mit Rotwein angeschmutzte gebleichte Baumwolle |
| Schmutzgewebe 3 | 2,5 Baumwollrohnesselgewebe |
| Spülen | 3 x 30 sec mit Leitungswasser (14° dH) |

Die Auswertung der Versuche wurde durch Remissionsmessungen an den getrockneten Geweben vorgenommen. Aus den an den einzelnen Prüfgeweben gemessenen Remissionswerten bei 18 Wellenlängen im Bereich von 400 bis 700 nm im Abstand von 20 nm wurden nach dem in A. Kud. Seifen, Öle, Fette, Wachse 119, S. 590 - 594 (1993) beschriebenen Verfahren die jeweiligen Farbstärken der Testanschmutzungen vor und nach der Wäsche bestimmt und daraus die absolute Bleichwirkung A_{abs} in % berechnet. In den nachfolgenden Tabellen ist die Bleichwirkung in Prozent angeben.

**Tabelle 1:**

| Testgewebe mit Grasanschmutzung | | | |
|---|---|---|---|
| Temperatur Bleichaktivator | 22°C | 38°C | 60°C |
| TAED | 27,2 | 32,0 | 41,7 |
| Beispiel 2 (konfektioniert) | 30,8 | 37,6 | 46,2 |

**Tabelle 2:**

| a) Chlorophyllanschmutzung | | | |
|---|---|---|---|
| Temperatur Bleichaktivator | 22°C | 38°C | 60°C |
| Octanoylcaprolactam | 31,1 | 39,5 | 45,6 |
| Beispiel 2 (konfektioniert) | 30,8 | 37,6 | 46,2 |

| b) Rotweinanschmutzung | | | |
|---|---|---|---|
| Temperatur Bleichaktivator | 22°C | 38°C | 60°C |
| Octanoylcaprolactam | 60,3 | 64,0 | 70,6 |
| Beispiel 2 (konfektioniert) | 63,8 | 67,6 | 73,9 |

| c) Baumwollrohnesselgewebe | | | |
|---|---|---|---|
| Temperatur Bleichaktivator | 22°C | 38°C | 60°C |
| Octanoylcaprolactam | 7,1 | 14,1 | 14,2 |
| Beispiel 2 (konfektioniert) | 9,1 | 13,9 | 19,6 |

Die Meßergebnisse aus den Waschversuchen in Tabelle 2 zeigen, daß mit der Konfektionierung, dargestellt am Beispiel von Octanoylcaprolactam, kein Wirkungsverlust in Kauf genommen werden muß.

## Patentansprüche

1. Homogen aufgebaute, feste Zusammensetzung aus im wesentlichen 5 bis 98 Gew.-Teilen heterocyclischer Verbindungen der allgemeinen Formel I
R¹―X―L¹ (I)
in der
L¹ für
(a) einen cyclischen Carbamatrest der Formel 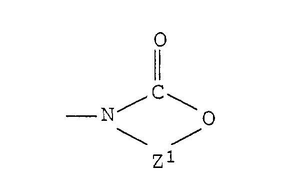
(b) einen Lactonoxyrest der Formel 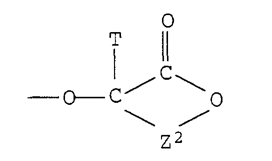 oder
(c) einen Lactamrest der Formel 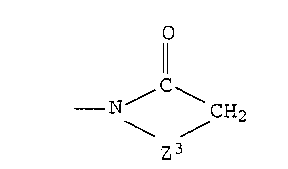 steht, wobei
Z¹ bis Z³ 1,2-, 1,3-, 1,4- oder 1,5-Alkylengruppen mit 2 bis 20 C-Atomen, welche zusätzlich durch ein bis drei Hydroxylgruppen, C₁- bis C₄-Alkoxygruppen, Aminogruppen, C₁- bis C₄-Alkylaminogruppen, Di-C₁- bis C₄-alkylaminogruppen, Chloratome, Bromatome, Nitrogruppen, Cyanogruppen, Carboxylgruppen, Sulfogruppen, Carboxy-C₁- bis C₄-alkylgruppen, Carboxamidgruppen oder Phenyl-, Tolyloder Benzylreste funktionalisiert, wobei aromatische Kerne ihrerseits ebenfalls durch die genannten Reste substituiert sein können, oder durch ein oder zwei nicht benachbarte Sauerstoffatome, Aminogruppen, C₁- bis C₄-Alkylaminogruppen oder Carbonylgruppen unterbrochen sein können, bezeichnen und
T Wasserstoff oder C₁- bis C₄-Alkyl bedeutet,
x eine sauerstoffhaltige Gruppe der Formel 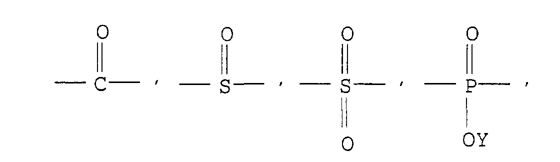 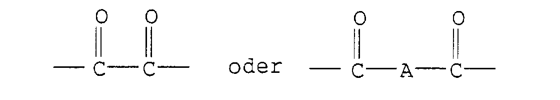 bedeutet, wobei
Y für Wasserstoff, Ammonium, welches gegebenenfalls durch organische Reste substituiert sein kann, oder C₁- bis C₄-Alkyl steht und
A eine chemische Bindung oder eine C₁- bis C₁₈-Alkylengruppe, eine C₂- bis C₁₈-Alkenylengruppe, eine C₅- bis C₃₂-Cycloalkylengruppe, eine C₇- bis C₃₀-Aralkylengruppe, eine C₆- bis C₁₈-Arylengruppe oder eine C₃- bis C₁₈-Heteroarylengruppe bezeichnet, wobei aliphatische Struktureinheiten zusätzlich durch ein bis fünf Hydroxylgruppen, C₁- bis C₄-Alkoxygruppen, Aminogruppen, C₁- bis C₄-Alkylaminogruppen, Di-C₁- bis C₄-alkylaminogruppen, Chloratome, Bromatome, Nitrogruppen, Cyanogruppen, Carboxylgruppen, Sulfogruppen, Carboxy-C₁- bis C₄-alkylgruppen, Carboxamidgruppen oder Phenyl-, Tolyloder Benzylreste funktionalisiert, wobei aromatische, cycloaliphatische und heteroaromatische Struktureinheiten ebenfalls durch die genannten Reste substituiert sein können, oder durch ein bis acht nicht benachbarte Sauerstoffatome, Aminogruppen, C₁- bis C₄-Alkylaminogruppen oder Carbonylgruppen unterbrochen sein können, und
R¹ folgende Bedeutung aufweist:
C₁- bis C₃₀-Alkyl, C₂- bis C₃₀-Alkenyl, C₅- bis C₁₈-Cycloalkyl, C₇- bis C₁₈-Aralkyl, C₆- bis C₁₈-Aryl oder C₃- bis C₁₈-Heteroaryl, wobei aliphatische Reste zusätzlich durch ein bis fünf Hydroxylgruppen, C₁- bis C₄-Alkoxygruppen, Aminogruppen, C₁bis C₄-Alkylaminogruppen, Di-C₁- bis C₄-alkylaminogruppen, Chloratome, Bromatome, Nitrogruppen, Cyanogruppen, Carboxylgruppen, Sulfogruppen, Carboxy-C₁- bis C₄-alkylgruppen, Carboxamidgruppen oder Phenyl-, Tolyl- oder Benzylreste funktionalisiert, wobei aromatische, cycloaliphatische und heteroaromatische Struktureinheiten ebenfalls durch die genannten Reste substituiert sein können, oder durch ein bis acht nicht benachbarte Sauerstoffatome, Aminogruppen, C₁- bis C₄-Alkylaminogruppen oder Carbonylgruppen unterbrochen sein können,
oder
einen heterocyclischen Rest L¹,
und/oder Oximestern der allgemeinen Formel II 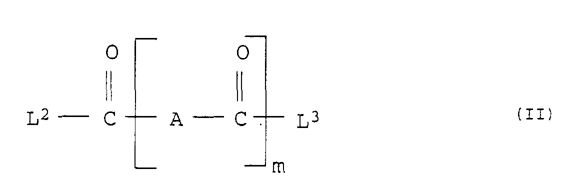 in der
L² für eine Oxim-Gruppierung der Formel 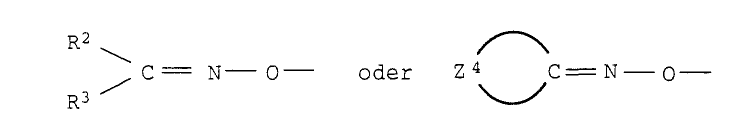 steht, wobei
R² und R³ Wasserstoff, C₁- bis C₃₀-Alkyl, C₂- bis C₃₀-Alkenyl, C₅- bis C₁₈-Cycloalkyl, C₇- bis C₁₈-Aralkyl oder C₆bis C₁₈-Aryl oder C₃- bis C₁₈-Heteroaryl, wobei aliphatische Reste zusätzlich durch ein bis fünf Hydroxylgruppen, C₁- bis C₄-Alkoxygruppen, Aminogruppen, C₁- bis C₄-Alkylaminogruppen, Di-C₁- bis C₄-alkylaminogruppen, Chloratome, Bromatome, Nitrogruppen, Cyanogruppen, Carboxylgruppen, Sulfogruppen, Carboxy-C₁- bis C₄-alkylgruppen, Carboxamidgruppen oder Phenyl-, Tolyl- oder Benzylreste funktionalisiert, wobei aromatische, cycloaliphatische und heteroaromatische Struktureinheiten ebenfalls durch die genannten Reste substituiert sein können, oder durch ein bis acht nicht benachbarte Sauerstoffatome, Aminogruppen, C₁- bis C₄-Alkylaminogruppen oder Carbonylgruppen unterbrochen sein können, bedeuten und
Z⁴ 1,3-, 1,4-, 1,5-, 1,6-, 1,7- oder 1,8-Alkylengruppen mit 3 bis 30 C-Atomen, welche zusätzlich durch ein bis fünf Hydroxylgruppen, C₁- bis C₄-Alkylaminogruppen, Di-C₁- bis C₄-alkylaminogruppen, Chloratome, Bromatome, Nitrogruppen, Cyanogruppen, Carboxylgruppen, Sulfogruppen, Carboxy-C₁- bis C₄-alkylgruppen, Carboxamidgruppen oder Phenyl-, Tolyl- oder Benzylreste funktionalisiert, wobei aromatische Kerne ihrerseits ebenfalls durch die genannten Reste substituiert sein können, oder durch ein oder zwei nicht benachbarte Sauerstoffatome, Aminogruppen, C₁- bis C₄-Alkylaminogruppen oder Carbonylgruppen unterbrochen sein können, bezeichnet,
L³ für den Rest R¹, eine zweite Oxim-Gruppierung L² oder für
(a) einen Carbonesterrest der Formel 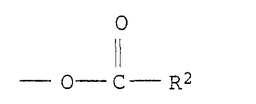
(b) einen Carbonamidrest der Formel 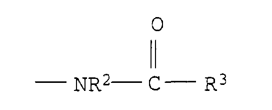
(c) einen Phenolatrest der Formel 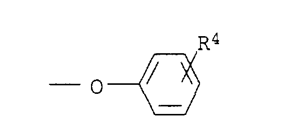
(d) einen Vinyloxyrest der Formel
―O― CR² = CHR³
(e) einen Sulfonamidrest der Formel 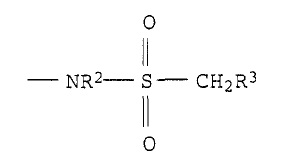
(f) einen Imidazolrest der Formel 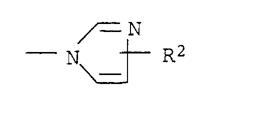
(g) einen Amidolactamrest der Formel 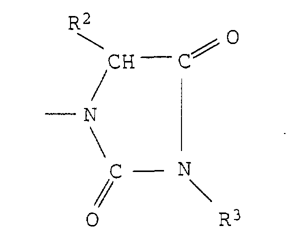
(h) einen cyclischen Carbamatrest der Formel 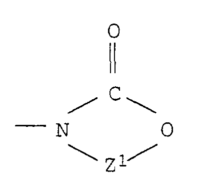
(j) einen Lactonoxyrest der Formel 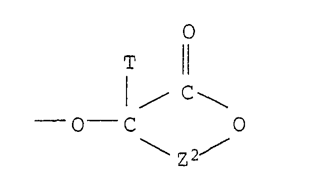 oder
(k) einen Lactamrest der Formel 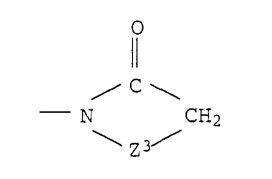 steht, wobei
R^{1,} R², R^{3,} T, Z¹ bis Z³ und A die oben genannten Bedeutungen haben,
R⁴ Wasserstoff, eine Carbonsäuregruppe, eine Sulfonsäuregruppe, eine Phosphonsäuregruppe oder deren Alkalimetall -oder Ammoniumsalz bedeutet und
m für die Zahl 0 oder 1 steht,
und 2 bis 95 Gew.-Teilen inerter poröser Trägermaterialien mit einer inneren Oberfläche von 10 bis 500 m²/g, erhältlich durch gleichmäßiges Einziehen der heterocyclischen Verbindungen I bzw. der Oximester II und gegebenenfalls weiterer Hilfsstoffe in die inneren Bereiche der Trägermaterial-Körner und dortiges gleichmäßiges Verteilen, dergestalt daß kein merkliches Konzentrationsgefälle der einziehenden Substanzen zwischen Innerem und äußerer Oberfläche der Körner oder Körner-Aggregate vorliegt.

2. Feste Zusammensetzung nach Anspruch 1, wobei die inerten porösen Trägermaterialien eine durchschnittliche Teilchengröße von 3 nm bis 2 mm aufweisen.

3. Feste Zusammensetzung nach Anspruch 1 oder 2, wobei als inerte poröse Trägermaterialien Kieselgele, Kieselsäuren, Aluminiumoxide, Kaoline und/oder Aluminiumsilicate eingesetzt werden.

4. Feste Zusammensetzung nach den Ansprüchen 1 bis 3, wobei die sauerstoffhaltige Gruppe X der heterocyclischen Verbindungen I 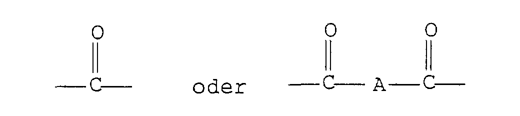 bedeutet.

5. Feste Zusammensetzung nach den Ansprüchen 1 bis 4, wobei der Rest R¹ in den heterocyclischen Verbindungen I C₆- bis C₁₈-Alkyl, C₆- bis C₁₈-Alkenyl, C₇- bis C₁₂-Aralkyl, Phenyl oder alkylsubstituiertes Phenyl mit insgesamt bis zu 14 C-Atomen oder einen zweiten heterocyclischen Rest L¹, der die gleiche Struktur wie der erste heterocyclische Rest L¹ aufweist, bedeutet.

6. Feste Zusammensetzung nach den Ansprüchen 1 bis 3, wobei der Rest L² der Oximester II für eine Oxim-Gruppierung der Formel 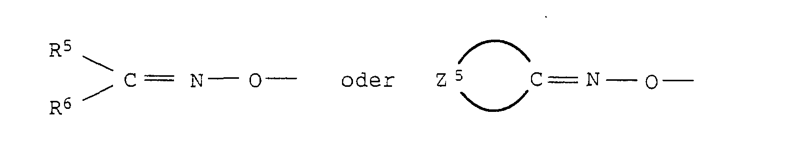 steht, wobei
R⁵ und R⁶ Wasserstoff, C₁- bis C₄-Alkyl, Phenyl oder Benzyl bedeutet und
Z⁵ 1,4-Butylen, 1,5-Pentylen oder 1,6-Hexylen bezeichnet.

7. Feste Zusammensetzung nach den Ansprüchen 1 bis 3 oder 6, wobei der Rest L³ der Oximester II für eine zweite Oxim-Gruppierung L² oder für einen C₁- bis C₁₈-Alkylrest, einen C₂- bis C₁₈-Alkenylrest, einen C₇- bis C₁₂-Aralkylrest oder Phenyl oder alkylsubstituiertes Phenyl mit insgesamt bis zu 14 C-Atomen steht.

8. Verfahren zur Herstellung einer festen Zusammensetzung gemäß den Ansprüchen 1 bis 7, **dadurch gekennzeichnet, daß** man 5 bis 98 Gew.-Teile heterocyclischer Verbindungen I und/oder Oximester II mit 2 bis 95 Gew.-Teilen inerter poröser Trägermaterialien durch gleichmäßiges Einziehen der Verbindungen I bzw. II und gegebenenfalls weiterer Hilfsstoffe in die inneren Bereiche der Trägermaterial-Körner und dortiges gleichmäßiges Verteilen miteinander mischt und diese Mischung gegebenenfalls durch übliche Methoden in eine für den jeweiligen Anwendungszweck geeignete konfektionierte Form bringt.

9. Verfahren zur Herstellung einer festen Zusammensetzung nach Anspruch 8, **dadurch gekennzeichnet, daß** man eine Lösung oder eine Schmelze der heterocyclischen Verbindungen I und/oder der Oximester II durch Einrühren, Aufsprühen oder Tränken mit den inerten porösen Trägermaterialien mischt, das Lösungsmittel, soweit vorhanden, durch Destillation oder Trocknen weitgehend entfernt und, wenn notwendig, die resultierende Mischung, gegebenenfalls nach Vermischen mit weiteren Hilfsstoffen, durch übliche Methoden in eine für den jeweiligen Anwendungszweck geeignete konfektionierte Form bringt.

10. Wasch-, Bleich- und Reinigungsmittel, enthaltend 0,1 bis 30 Gew.-%, bezogen auf die Gesamtmenge der Formulierung, der festen Zusammensetzung gemäß den Ansprüchen 1 bis 7.

11. Verwendung einer festen Zusammensetzung gemäß den Ansprüchen 1 bis 7 als stabile Bleichaktivator-Komponente in Wasch-, Bleich- und Reinigungsmitteln.

## Claims

1. A homogeneously composed, solid composition consisting essentially of 5-98 parts by weight of heterocyclic compounds of the general formula I
R¹―X―L¹ (I)
where
L¹ is
(a) a cyclic carbamate residue of the formula 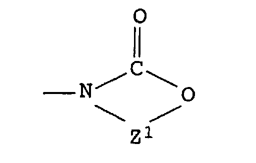
(b) a lactonoxy residue of the formula 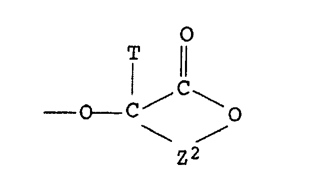 or
(c) a lactam residue of the formula 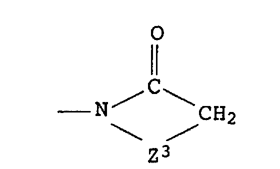 where
Z¹ to Z³ are 1,2-, 1,3-, 1,4- or 1,5-alkylene groups which have 2 to 20 carbon atoms, and which can additionally be functionalized by one to three hydroxyl groups, C₁-C₄-alkoxy groups, amino groups, C₁-C₄-alkylamino groups, di-C₁-C₄-alkylamino groups, chlorine atoms, bromine atoms, nitro groups, cyano groups, carboxyl groups, sulfo groups, carboxy-C₁-C₄-alkyl groups, carboxamide groups or phenyl, tolyl or benzyl radicals, it likewise being possible for aromatic nuclei in turn to be substituted by said radicals, or interrupted by one or two non-adjacent oxygen atoms, amino groups, C₁-C₄-alkylamino groups or carbonyl groups, and
T is hydrogen or C₁-C₄-alkyl,
X is an oxygen-containing group of the formula 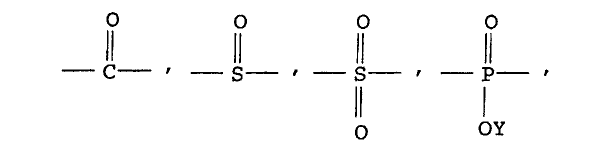 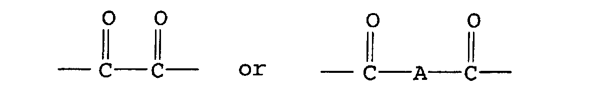 where
Y is hydrogen, ammonium which can be unsubstituted or substituted by organic radicals, or C₁-C₄-alkyl, and
A is a chemical bond or a C₁-C₁₈-alkylene group, a C₂-C₁₈-alkenylene group, a C₅-C₃₂-cycloalkylene group, a C₇-C₃₀-aralkylene group, a C₆-C₁₈-arylene group or a C₃-C₁₈-hetarylene group, it additionally being possible for aliphatic structural units to be functionalized by one to five hydroxyl groups, C₁-C₄-alkoxy groups, amino groups, C₁-C₄-alkylamino groups, di-C₁-C₄-alkylamino groups, chlorine atoms, bromine atoms, nitro groups, cyano groups, carboxyl groups, sulfo groups, carboxy-C₁-C₄-alkyl groups, carboxamide groups or phenyl, tolyl or benzyl radicals, it likewise being possible for aromatic, cycloaliphatic and heteroaromatic structural units to be substituted by said radicals, or interrupted by one to eight non-adjacent oxygen atoms, amino groups, C₁-C₄-alkylamino groups or carbonyl groups, and
R¹ has the following meaning:
C₁-C₃₀-alkyl, C₂-C₃₀-alkenyl, C₅-C₁₈-cycloalkyl, C₇-C₁₈-aralkyl, C₆-C₁₈-aryl or C₃-C₁₈-hetaryl, it being additionally possible for aliphatic radicals to be functionalized by one to five hydroxyl groups, C₁-C₄-alkoxy groups, amino groups, C₁-C₄-alkylamino groups, di-C₁-C₄-alkylamino groups, chlorine atoms, bromine atoms, nitro groups, cyano groups, carboxyl groups, sulfo groups, carboxy-C₁-C₄-alkyl groups, carboxamide groups or phenyl, tolyl or benzyl radicals, it likewise being possible for aromatic, cycloaliphatic and heteroaromatic structural units to be substituted by said radicals, or interrupted by one to eight non-adjacent oxygen atoms, amino groups, C₁-C₄-alkylamino groups or carbonyl groups,
or
a heterocyclic radical L¹,
and/or oxime esters of the general formula II 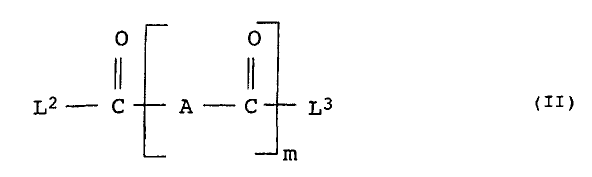 where
L² is an oxime moiety of the formula 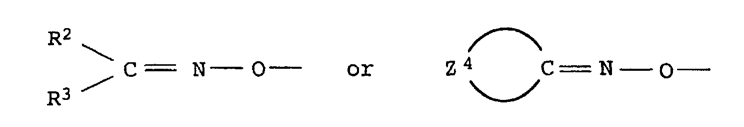 where
R² and R³ are hydrogen, C₁-C₃₀-alkyl, C₂-C₃₀-alkenyl, C₅-C₁₈-cycloalkyl, C₇-C₁₈-aralkyl or C₆-C₁₈-aryl or C₃-C₁₈-hetaryl, it being additionally possible for aliphatic radicals to be functionalized by one to five hydroxyl groups, C₁-C₄-alkoxy groups, amino groups, C₁-C₄-alkylamino groups, di-C₁-C₄-alkylamino groups, chlorine atoms, bromine atoms, nitro groups, cyano groups, carboxyl groups, sulfo groups, carboxy-C₁-C₄-alkyl groups, carboxamide groups or phenyl, tolyl or benzyl radicals, it likewise being possible for aromatic, cycloaliphatic and heteroaromatic structural units to be substituted by said radicals, or interrupted by one to eight non-adjacent oxygen atoms, amino groups, C₁-C₄-alkylamino groups or carbonyl groups, and
Z⁴ is 1,3-, 1,4-, 1,5-, 1,6-, 1,7- or 1,8-alkylene groups which have 3 to 30 carbon atoms and which can additionally be functionalized by one to five hydroxyl groups, C₁-C₄-alkylamino groups, di-C₁-C₄-alkylamino groups, chlorine atoms, bromine atoms, nitro groups, cyano groups, carboxyl groups, sulfo groups, carboxy-C₁-C₄-alkyl groups, carboxamide groups or phenyl, tolyl or benzyl radicals, it likewise being possible for aromatic nuclei in turn to be substituted by said radicals, or interrupted by one or two non-adjacent oxygen atoms, amino groups, C₁-C₄-alkylamino groups or carbonyl groups,
L³ is the radical R¹, a second oxime moiety L² or
(a) a carboxylic ester residue of the formula 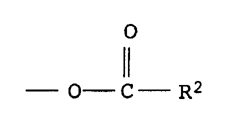
(b) a carboxamide residue of the formula 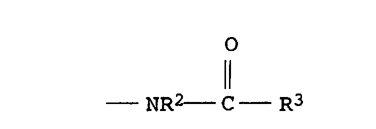
(c) a phenolate residue of the formula 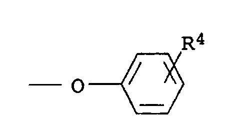
(d) a vinyloxy radical of the formula
― O― CR² = CHR³
(e) a sulfonamide residue of the formula 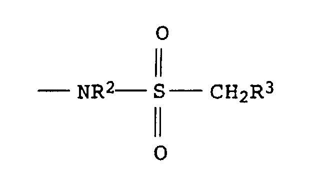
(f) an imidazole residue of the formula 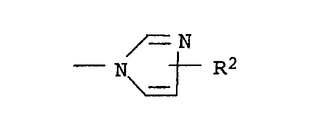
(g) an amidolactam residue of the formula 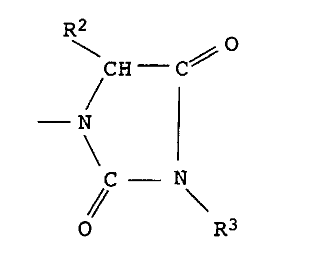
(h) a cyclic carbamate residue of the formula 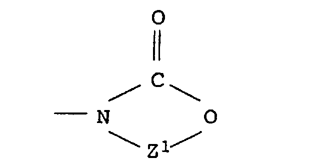
(j) a lactonoxy residue of the formula 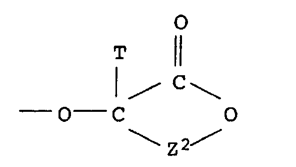 or
(k) a lactam residue of the formula 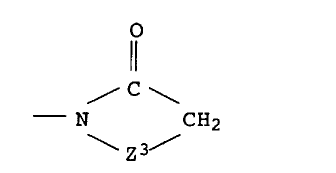
where
R^{1,} R², R^{3,} T, Z¹ to Z³ and A have the abovementioned meanings,
R⁴ is hydrogen, a carboxyl group, a sulfo group, a phosphono group or the alkali metal or ammonium salt thereof, and
m is the number 0 or 1,
and 2-95 parts by weight of inert porous carrier materials with an internal surface area of from 10 to 500 m²/g, obtainable by the heterocyclic compounds I and the oxime esters II and, where appropriate, other auxiliaries being uniformly drawn into the interior of the carrier material particles and uniformly distributed therein in such a way that there is no noticeable gradient of concentration of the substances which are drawn in between the inner and outer surface of the particles or particle aggregates.

2. A solid composition as claimed in claim 1, wherein the inert porous carrier materials have an average particle size of from 3 nm to 2 mm.

3. A solid composition as claimed in claim 1 or 2, wherein silica gels, silicas, aluminum oxide, kaolins and/or aluminum silicates are employed as inert porous carrier materials.

4. A solid composition as claimed in any of claims 1 to 3, wherein the oxygen-containing group X in the heterocyclic compounds I is

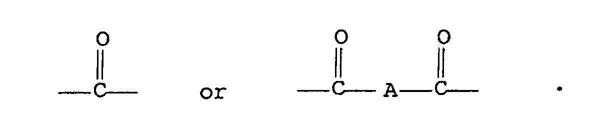

5. A solid composition as claimed in any of claims 1 to 4, wherein the radical R¹ in the heterocyclic compounds I is C₆-C₁₈-alkyl, C₆-C₁₈-alkenyl, C₇-C₁₂-aralkyl, phenyl or alkyl-substituted phenyl with a total of up to 14 carbon atoms or a second heterocyclic radical L¹ which has the same structure as the first heterocyclic radical L¹.

6. A solid composition as claimed in any of claims 1 to 3, wherein the radical L² in the oxime esters II is an oxime moiety of the formula 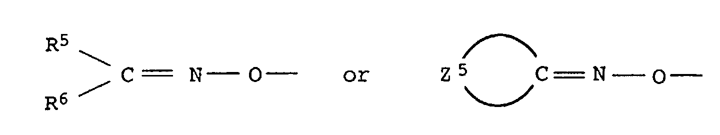 where
R⁵ and R⁶ are hydrogen, C₁-C₄-alkyl, phenyl or benzyl, and
Z⁵ is 1,4-butylene, 1,5-pentylene or 1,6-hexylene.

7. A solid composition as claimed in any of claims 1 to 3 or 6, wherein the radical L³ in the oxime esters II is a second oxime moiety L² or a C₁-C₁₈-alkyl radical, a C₂-C₁₈-alkenyl radical, a C₇-C₁₂-aralkyl radical or phenyl or alkyl-substituted phenyl with a total of up to 14 carbon atoms.

8. A process for preparing a solid composition as claimed in any of claims 1 to 7, which comprises mixing together 5-98 parts by weight of heterocyclic compounds I and/or oxime esters II with 2-95 parts by weight of inert porous carrier materials by the compounds I and II and, where appropriate, other auxiliaries being uniformly drawn into the interior of the carrier material particles and uniformly distributed therein, and converting this mixture where appropriate by conventional methods into a formulated form suitable for the particular application.

9. A process for preparing a solid composition as claimed in claim 8, wherein a solution or a melt of the heterocyclic compounds I and/or of the oxime esters II is mixed with the inert porous carrier materials by stirring in, spraying on or impregnating, the solvent, where present, is substantially removed by distillation or drying and, if necessary, the resulting mixture is converted, where appropriate after mixing with other auxiliaries, by conventional methods into a formulated form suitable for the particular application.

10. A detergent, bleach or cleaner comprising 0.1-30% by weight, based on the total amount of the formulation, of the solid composition as claimed in any of claims 1 to 7.

11. The use of a solid composition as claimed in any of claims 1 to 7 as stable bleach activator component in detergents, bleaches and cleaners.

## Revendications

1. Composition solide, ayant une constitution homogène constituée essentiellement de 5 à 98 parties en poids de composés hétérocycliques de formule générale I
R¹-X-L¹ (I)
dans laquelle
L¹ est mis pour
(a) un reste carbamate cyclique de formule 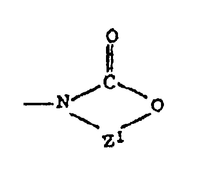
(b) un reste lactone-oxy de formule 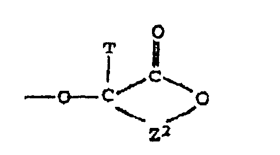 ou
(c) un reste lactame de formule 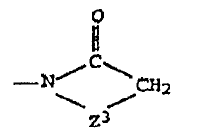 dans lesquelles
Z¹ à Z³ représentent des groupes 1,2-, 1,3-, 1,4- ou 1,5-alkylène ayant 2 à 20 atomes de C, lesquels sont fonctionnalisés de plus par un à trois groupes hydroxyle, alcoxy en C₁ à C₄, amino, alkylamino en C₁ à C₄, di-(alkyle en C₁ à C₄)-amino, atomes de chlore, de brome, groupes nitro, cyano, carboxyle, sulfo, carboxy-(alkyle en C₁ à C₄), carboxamide ou restes phényle, tolyle ou benzyle, les noyaux aromatiques pouvant être de leur côté substitués également par les restes cités, ou pouvant être interrompus par un ou deux atomes d'oxygène non voisins, groupes amino, (alkyle en C₁ à C₄)-amino ou carbonyle, et
T représente un atome d'hydrogène ou un groupe alkyle en C₁ à C₄,
X représente un groupe contenant de l'oxygène de formule 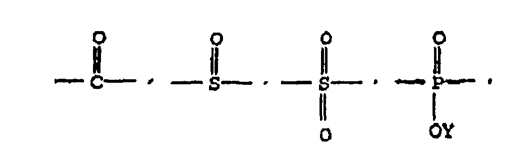 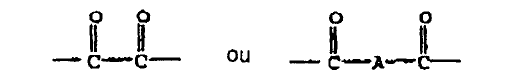 où
Y est mis pour un atome d'hydrogène, un groupe ammonium, qui peut être substitué éventuellement par des restes organiques, ou un groupe alkyle en C₁ à C₄ et
A désigne une liaison chimique ou un groupe alkylène en C₁ à C₁₈, un groupe alcénylène en C₂ à C₁₈, un groupe cycloalkylène en C₅ à C₃₂, un groupe aralkylène en C₇ à C₃₀, un groupe arylène en C₆ à C₁₈ ou un groupe hétéroarylène en C₃ à C₁₈, les motifs structuraux aliphatiques pouvant être fonctionnalisés de plus par un à cinq groupes hydroxyle, alcoxy en C₁ à C₄, amino, (alkyle en C₁ à C₄)-amino, di-(alkyle en C₁ à C₄)-amino, atomes de chlore, de brome, groupes nitro, cyano, carboxyle, sulfo, carboxy-(alkyle en C₁ à C₄), carboxamide ou restes phényle, tolyle ou benzyle, les motifs structuraux aromatiques, cycloaliphatiques et hétéroaromatiques pouvant être également substitués par les restes cités, ou pouvant être interrompus par un à huit atomes d'oxygène non voisins, groupes amino, (alkyle en C₁ à C₄)-amino ou carbonyle, et
R¹ prend la signification suivante:
un groupe alkyle en C₁ à C₃₀, alcényle en C₂ à C₃₀, cycloalkyle en C₅ à C₁₈, aralkyle en C₇ à C₁₈, aryle en C₆ à C₁₈ ou hétéroaryle en C₃ à C₁₈, les restes aliphatiques pouvant être de plus fonctionnalisés par 1 à 5 groupes hydroxyle, alcoxy en C₁ à C₄, amino, (alkyle en C₁ à C₄)-amino, di-(alkyle en C₁ à C₄)-amino, atomes de chlore, de brome, groupes nitro, cyano, carboxyle, groupes sulfo, carboxy-(alkyle en C₁ à C₄), carboxamide ou restes phényle, tolyle ou benzyle, les motifs structuraux aromatiques, cycloaliphatiques et hétéroaromatiques pouvant être substitués également par les restes cités, ou pouvant être interrompus par un à huit atomes d'oxygène non voisins, groupes amino, (alkyle en C₁ à C₄)-amino ou carbonyle,
ou
un reste hétérocyclique L¹,
et/ou des esters d'oxime de formule générale II 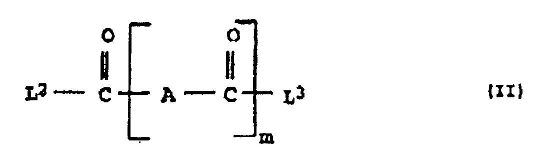 dans laquelle
L² est mis pour un groupement oxime de formule 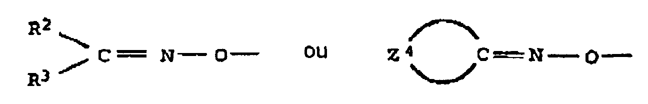
dans lesquelles
R² et R³ représentent un atome d'hydrogène, un groupe alkyle en C₁ à C₃₀, alcényle en C₂ à C₃₀, cycloalkyle en C₅ à C₁₈, aralkyle en C₇ à C₁₈ ou aryle en C₆ à C₁₈ ou hétéroaryle en C₃ à C₁₈, les restes aliphatiques pouvant être fonctionnalisés de plus par 1 à 5 groupes hydroxyle, alcoxy en C₁ à C₄, amino, (alkyle en C₁ à C₄)-amino, di-(alkyle en C₁ à C₄)-amino, atomes de chlore, de brome, groupes nitro, cyano, carboxyle, sulfo, carboxy-(alkyle en C₁ à C₄), carboxamide ou restes phényle, tolyle ou berizyle, les motifs structuraux aromatiques, cycloaliphatiques et hétéroaromatiques pouvant être substitués également par les restes cités, ou pouvant être interrompus par un à huit atomes d'oxygène non voisins, groupes amino, (alkyle en C₁ à C₄)-amino, ou carbonyle, et
Z⁴ représente des groupes 1,3-, 1,4-, 1,5-, 1,6-, 1,7- ou 1,8-alkylène ayant 3 à 30 atomes de C, lesquels sont en outre fonctionnalisés par un à cinq groupes hydroxyle, (alkyle en C₁ à C₄)-amino, di-(alkyle en C₁ à C₄)-amino, atomes de chlore, de brome, groupes nitro, cyano, carboxyle, sulfo, carboxy-(alkyle en C₁ à C₄), carboxamide ou restes phényle, tolyle, ou benzyle, les noyaux aromatiques pouvant être substitués de leur côté également par les restes cités, ou pouvant être interrompus par un ou deux atomes d'oxygène non voisins, groupes amino, (alkyle en C₁ à C₄)-amino ou carbonyle,
L³ est mis pour le reste R¹, un deuxième groupement oxime L² ou pour
(a) un reste d'ester carboxylique de formule 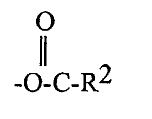
(b) un reste d'amide carboxylique de formule 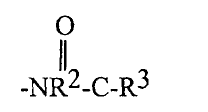
(c) un reste phénolate de formule 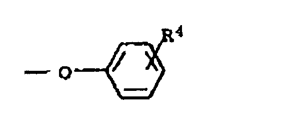
(d) un reste vinyloxy de formule
-O-CR²=CHR³
(e) un reste sulfonamide de formule 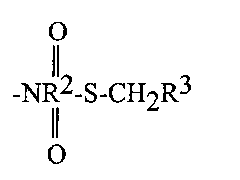
(f) un reste imidazole de formule 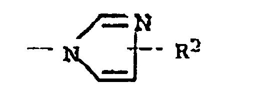
(g) un reste amidolactame de formule 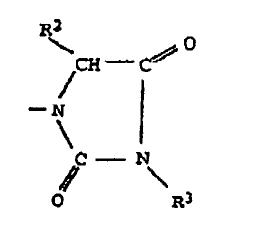
(h) un reste carbamate cyclique de formule 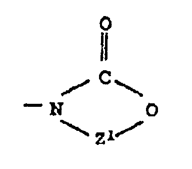
(j) un reste lactone-oxy de formule 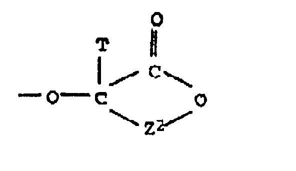 ou
(k) un reste lactame de formule 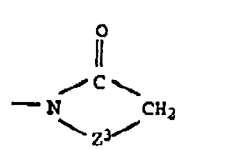 dans lesquelles
R¹, R², R³, T, Z¹ à Z³ et A prennent les significations précitées,
R⁴ représente un atome d'hydrogène, un groupe acide carboxylique, un groupe acide sulfonique, un groupe acide phosphonique ou leur sel de métal alcalin ou d'ammonium et
m est mis pour le nombre 0 ou 1,
et 2 à 95 parties en poids de matériaux support, inertes et poreux ayant une surface interne de 10 à 500 m²/g, que l'on peut obtenir en insérant dans des conditions homogènes des composés hétérocycliques I ou des esters d'oxime II et éventuellement d'autres adjuvants dans les domaines intérieurs des grains du matériau support, et en les y répartissant d'une manière homogène, de telle sorte qu'aucun gradient marqué de concentration des substances à introduire n'apparaisse entre la surface intérieure et la surface extérieure des grains ou des agrégats de grains.

2. Composition solide selon la revendication 1, dans laquelle les matériaux support, poreux et inertes présentent une granulométrie moyenne de 3 nm à 2 mm.

3. Composition solide selon la revendication 1 ou 2, dans laquelle des gels de silice, des acides siliciques, des oxydes d'aluminium, des kaolins et/ou des silicates d'aluminium sont mis en oeuvre en tant que matériaux support, poreux et inertes.

4. Composition solide selon les revendications 1 à 3, dans laquelle le groupe X contenant de l'oxygène des composés hétérocycliques I représente

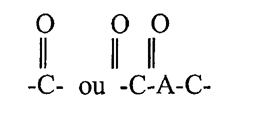

5. Composition solide selon les revendications 1 à 4, dans laquelle le reste R¹ dans les composés hétérocycliques I, représente un groupe alkyle en C₆ à C₁₈, alcényle en C₆ à C₁₈, aralkyle en C₇ à C₁₂, phényle ou phényle à substitution alkyle ayant au total jusqu'à 14 atomes de C ou un deuxième reste hétérocyclique L¹, qui présente la même structure que le premier reste hétérocyclique L¹.

6. Composition solide selon les revendications 1 à 3, dans laquelle le reste L² des esters d'oxime II est mis pour un groupement oxime de formule 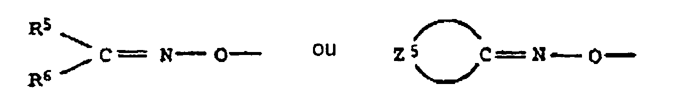 dans lesquelles
R⁵ et R⁶ représentent un atome d'hydrogène, un groupe alkyle en C₁ à C₄, phényle ou benzyle, et
Z⁵ désigne un groupe 1,4-butylène, 1,5-pentylène ou 1,6-hexylène.

7. Composition solide selon les revendications 1 à 3 ou 6, dans laquelle le reste L³ des esters d'oxime II est mis pour un deuxième groupement oxime L² ou pour un reste alkyle en C₁ à C₁₈, un reste alcényle en C₂ à C₁₈, un reste aralkyle en C₇ à C₁₂ ou phényle ou phényle à substitution alkyle ayant au total jusqu'à 14 atomes de C.

8. Procédé de préparation d'une composition solide selon les revendications 1 à 7, **caractérisé en ce que** l'on mélange d'une façon habituelle entre elles, 5 à 98 parties en poids des composés hétérocycliques I et/ou des esters d'oxime II avec 2 à 95 parties en poids de matériaux support, inertes et poreux, en introduisant dans des conditions homogènes les composés I ou II et éventuellement d'autres adjuvants dans les domaines intérieurs des grains du matériau support et en les y répartissant d'une manière homogène,et que l'on confère à ce mélange, éventuellement à l'aide de procédés habituels, une forme confectionnée et adaptée aux buts d'utilisation respectifs.

9. Procédé de préparation d'une composition solide selon la revendication 8, **caractérisé en ce que** l'on mélange une solution ou une masse fondue des composés hétérocycliques I et/ou des esters d'oxime II à l'aide d'une agitation, d'une pulvérisation ou d'une imprégnation avec les matériaux support, inertes et poreux, **en ce que** l'on élimine en grande partie le solvant, s'il y en a, au moyen d'une distillation ou d'un séchage et, si nécessaire, on confère au mélange résultant, éventuellement après mélange avec des adjuvants supplémentaires, à l'aide de procédés habituels, une forme confectionnée, adaptée aux buts d'utilisation respectifs.

10. Agent de lavage, de blanchiment et de nettoyage, contenant 0,1 à 30% en poids, par rapport à la quantité totale de la formulation, de la composition solide selon les revendications 1 à 7.

11. Utilisation d'une composition solide selon les revendications 1 à 7 en tant que composant stable d'activation de blanchiment dans des agents de lavage, de blanchiment et de nettoyage.
